# EUROPEAN PATENT APPLICATION

(11) **EP 3 761 039 A1**
(43) Date of publication of application: **06.01.2021**
(21) Application number: 19760299.8
(22) Date of filing: 28.01.2019
(51) Int. Cl.: G01N 35/04

(54) **BF SEPARATING DEVICE, SAMPLE ANALYZING DEVICE, AND BF SEPARATING METHOD**

(30) Priority: 28.02.2018 JP 2018034671
(71) Applicant: SYSMEX CORPORATION, Kobe-shi Hyogo 651-0073 (JP)
(72) Inventor: OISHI, Naoki, Kobe-shi, Hyogo 651-0073 (JP); MATSUMOTO, Yusuke, Kobe-shi, Hyogo 651-0073 (JP)
(74) Representative: Hoffmann Eitle
(86) International application number: PCT/JP2019/002757
(87) International publication number: WO 2019/167514

(57) **Abstract**

The degree of freedom in device configuration is increased. ABF separator includes: a holder configured to hold a container that stores therein a sample containing magnetic particles and a liquid component; a magnet provided to be movable relative to the holder in a direction approaching or separating from the holder, and configured to magnetically collect the magnetic particles; a movement mechanism configured to move the holder, and move the magnet in conjunction with movement of the holder, in a direction in which the holder and the magnet approach each other or separate from each other; an agitator configured to agitate the sample in the container held by the holder located in a separate position where the holder and the magnet are away from each other; and a suction tube configured to suck the liquid component in the container held by the holder located in an approach position where the holder and the magnet are close to each other.

## Description

### TECHNICAL FIELD

The present invention relates to a BF separator, a sample analyzer, and a BF separation method.

### BACKGROUND ART

In sample analysis using a sample analyzer, it is known that BF separation is performed to separate a solid phase (Bound) bound to a test substance from a liquid component containing a substance (Free) that is not bound to the test substance and therefore is free (see PATENT LITERATURE 1, for example).

PATENT LITERATURE 1 discloses, as shown in FIG. 22, a magnetic separation agitation device including: a reaction container 901 in which a reaction liquid is stored; a base 903 having a container setting portion 902 which is a recess for holding the reaction container 901; ferromagnetic bodies 905 disposed in cavities 904 which radially extend from a position, in the base 903, close to the container setting part 902; and a drive means 906 which rotates the base 903 around a vertical central axis at which the container setting part 902 is disposed. In this magnetic separation agitation device, the reaction container 901 is gripped by a catcher and set in the container setting part 902. Each ferromagnetic body 905 is urged toward the position close to the container setting part 902 in the cavity 904, and when a centrifugal force caused by rotation of the base 903 exceeds the urging force, the ferromagnetic body 905 moves to a position away from the reaction container 901 in the cavity 904.

The device disclosed in PATENT LITERATURE 1 stops the rotation of the base 903, and magnetically collects magnetic particles (solid phase) in the reaction container 901 by the magnetic force of the ferromagnetic bodies 905 close to the container setting part 902. The liquid component in the reaction container 901 is sucked by a nozzle 907 with the magnetic particles being magnetically collected by the ferromagnetic bodies 905, thereby separating the magnetic particles from the liquid component. Then, the device disclosed in PATENT LITERATURE 1 discharges a washing liquid into the reaction container 901 by using the nozzle 907, and thereafter separates the ferromagnetic bodies 905 from the reaction container 901 with a predetermined centrifugal force generated by rotation of the base 903, whereby the magnetic field near the reaction container 901 is varied to agitate the sample in the reaction container 901.

### CITATION LIST

### [PATENT LITERATURE]

[PTL 1] Japanese Patent No. 6063761

### SUMMARY OF THE INVENTION

### PROBLEMS TO BE SOLVED BY THE INVENTION

In the device disclosed in PATENT LITERATURE 1, the BF separation process (magnetic collection, insertion of the nozzle into the reaction container, suction, and agitation) needs to be performed in the container setting position where the reaction container is set. Therefore, for example, there is a restriction on the device configuration such as the device needing to be designed in a way that prevents interference between the catcher and the nozzle in a space above the container setting position. In addition, the device disclosed in PATENT LITERATURE 1 needs to be provided with a high-power drive source that generates a great centrifugal force for moving the ferromagnetic bodies. Such a drive source is likely to reduce the degree of freedom in the device configuration.

### SOLUTION TO THE PROBLEMS

A first aspect of the present invention relates to a BF separator including: a holder (10) configured to hold a container (90) which stores therein a sample containing magnetic particles and a liquid component; a magnet (20) provided to be movable relative to the holder (10) in a direction approaching or separating from the holder (10), and configured to magnetically collect the magnetic particles; a movement mechanism (30) configured to move the holder (10), and move the magnet (20) in conjunction with movement of the holder (10), in a direction in which the holder (10) and the magnet (20) approach each other or separate from each other; an agitator (40) configured to agitate the sample in the container (90) held by the holder (10) located in a separate position (62) where the holder (10) and the magnet (20) are away from each other; and a suction tube (50) configured to suck the liquid component in the container (90) held by the holder (10) located in an approach position (61) where the holder (10) and the magnet (20) are close to each other.

Since the holder (10) and the magnet (20) move in conjunction with one another, each of the approach position and the separate position is a conception that defines the positions of both the holder (10) and the magnet (20). That is, when the holder (10) and the magnet (20) are in the approach position, the positional relationship between them is different from that when the holder (10) and the magnet (20) are in the separate position. The holder (10) and the magnet (20) are closer to each other in the approach position than in the separate position. When the holder (10) and the magnet (20) are in the separate position, the positional relationship between them is different from that when the holder (10) and the magnet (20) are in the approach position. The holder (10) and the magnet (20) are farther from each other in the separate position than in the approach position. The holder (10) in the approach position and the magnet (20) in the approach position are not spatially in the same position. The holder (10) in the separate position and the magnet (20) in the separate position are not spatially in the same position.

As described above, the BF separator according to the first aspect includes the movement mechanism (30) which, in conjunction with movement of the holder (10), moves the magnet (20) in the direction in which the holder (10) and the magnet (20) approach each other or separate from each other. Therefore, by only moving the holder (10) in which the container (90) is set, the holder (10) and the magnet (20) can be moved to the separate position (62) where they are away from each other and to the approach position (61) where they are close to each other. Therefore, the BF separation process (magnetic collection, insertion of the suction tube, suction, and agitation) can be performed in a position different from the container setting position. In addition, with the simple configuration, the container (90) and the magnet (20) can be moved to the positional relationship for the magnetic collection, and the container (90) and the magnet (20) can be moved to the positional relationship for the agitation. Consequently, the degree of freedom in the device configuration can be increased.

In the BF separator according to the first aspect, preferably, the approach position (61) and the separate position (62) are different positions in a height direction. With this configuration, magnetic collection in the approach position (61) and agitation in the separate position (62) can be performed at different positions in the up-down direction. Therefore, as compared to the case where the approach position (61) and the separate position (62) are arranged in a horizontal plane, the movement distance of the holder (10) in the horizontal direction between the approach position (61) and the separate position (62) can be reduced by the difference in height, and thus the installation area of the BF separator in the horizontal direction can be reduced.

Preferably, the BF separator according to the first aspect further includes a discharge tube (151) configured to discharge a washing liquid into the container (90) held by the holder (110) located in the approach position (61). The agitator (140) agitates the sample, in the container (90), containing the magnetic particles (82) and the washing liquid. With this configuration, both suction of the liquid component by the suction tube (150) and discharge of the washing liquid by the discharge tube (151) can be performed in the approach position (61), and therefore, the device configuration can be simplified as compared to the case where suction of the liquid component and discharge of the washing liquid are performed in different positions.

In this case, preferably, the movement mechanism (130) moves the holder (110) and the magnet (120) to the approach position (61) and to the separate position (62), a predetermined number of times, so as to repeat: suction of the liquid component by the suction tube (150) and discharge of the washing liquid by the discharge tube (151) in the approach position (61); and agitation of the sample by the agitator (140) in the separate position (62). With this configuration, suction of the liquid component and discharge of the washing liquid, and agitation of the sample can be repeated the plurality of number of times, thereby enhancing separation performance in the BF separation process. Even in the case where suction of the liquid component and discharge of the washing liquid, and agitation of the sample are repeated the plurality of number of times, it is only necessary to cause the magnet (120) and the holder (110) holding the container (90) to reciprocate between the approach position (61) and the separate position (62). Since an operation to take out the container (90) from the holder (110) is not required, the device configuration is simplified, and the BF separation process can be performed in a short time.

In the BF separator according to the first aspect, preferably, in the approach position (61), the holder (110) and the magnet (120) are close to each other over a distance that allows the magnetic particles (82) in the container (90) to be magnetically collected. In the separate position (62), the holder (110) and the magnet (120) are away from each other over a distance that allows magnetic collection of the magnetic particles (82) in the container (90) to be canceled. With this configuration, when suction of the liquid component by the suction tube (150) is performed in the approach position (61), suction of the magnetic particles (82) in the container (90) together with the liquid component can be effectively inhibited by the magnetic collection. In addition, since the magnetic collection of the magnetic particles (82) is canceled when the sample is agitated by the agitator (140) in the separate position (62), an unnecessary substance adhered to the magnetic particles (82) can be effectively dispersed in the liquid component.

In the BF separator according to the first aspect, preferably, the agitator (140) is connected to the holder (110) so as to move together with the holder (110), and configured to agitate the sample in the container (90) by driving the holder (110). With this configuration, the agitator (140) can be moved integrally with the holder (110) by the movement mechanism (130). Therefore, the device configuration can be simplified as compared to, for example, a configuration in which the holder (110) is connected to a fixed agitator (140) when moving to the separate position (62).

In the BF separator according to the first aspect, preferably, the movement mechanism (130) is configured to move the holder (110) and the magnet (120) in an up-down direction, and the holder (110) and the magnet (120) relatively move so as to approach each other or separate from each other in a direction intersecting the up-down direction, in conjunction with the movement in the up-down direction. With this configuration, the movement mechanism (130) only has to simply move the holder (110) and the magnet (120) in the up-down direction, and need not move them in the horizontal direction. Therefore, the configuration of the movement mechanism (130) can be simplified, and the installation area of the movement mechanism (130) can be reduced.

Preferably, the BF separator according to the first aspect further includes a guide portion (170) configured to come into contact with at least one of the holder (110) and the magnet (120) which are moved by the movement mechanism (130), and cause the holder (110) and the magnet (120) to approach each other or separate from each other. With this configuration, using movements of the holder (110) and the magnet (120) to the approach position (61) and to the separate position (62) by the movement mechanism (130), the holder (110) and the magnet (120) can be caused to approach each other or separate from each other by the contact with the guide portion (170) during the movement. Therefore, it is not necessary to provide a drive source for relatively moving the holder (110) and the magnet (120), in addition to the movement mechanism (130) for moving them between the approach position (61) and the separate position (62). Thus, the device configuration can be simplified.

In this case, preferably, the BF separator further includes a biasing member (125) configured to bias the holder (110) and the magnet (120) in a direction in which the holder and the magnet approach each other. The guide portion (170) is configured to cause the holder (110) and the magnet (120) to separate from each other against a biasing force of the biasing member (125), and cause the holder (110) and the magnet (120) to approach each other by the biasing force of the biasing member (125). With this configuration, the guide portion (170) only has to be formed to separate the holder (110) and the magnet (120) from each other against the urging force of the urging member (125), and it is not necessary to provide both a guide for causing the holder (110) and the magnet (120) to separate from each other and a guide for causing the holder (110) and the magnet (120) to approach each other. Therefore, the configuration of the guide portion (170) can be simplified.

In the configuration having the guide portion (170), preferably, the guide portion (170) has a surface (171) that is inclined with respect to a movement direction from the approach position (61) to the separate position (62) such that the holder (110) and the magnet (120) are separated from each other as the guide portion approaches from the approach position (61) to the separate position (62). At least one of the holder (110) and the magnet (120) includes a rotating body (121) which rolls on the inclined surface (171). With this configuration, when the rotating body (121) rolls on the inclined surface (171) of the guide portion (170), at least one of the holder (110) and the magnet (120) can be relatively moved in a direction different from the direction of movement from the approach position (61) to the separate position (62) according to the movement mechanism (130). As a result, the holder (110) and the magnet (120) can be made to smoothly approach or separate from each other by the simple configuration including the inclined surface (171) and the rotating body (121).

In the BF separator according to the first aspect, preferably, the magnet (120) is disposed laterally to the holder (110) so as to approach or separate from a side surface of the container (90) held by the holder (110). With this configuration, the magnetic particles (82) can be magnetically collected on the side surface of the container (90) in the approach position (61). Therefore, when the liquid component is sucked by the suction tube (150), the liquid component on the bottom surface of the container (90) can be satisfactorily sucked without sucking the collected magnetic particles (82), whereby the magnetic particles (82) can be effectively separated from the liquid component.

In this case, preferably, the holder (110) is configured to expose the side surface, on the magnet (120) side, of the held container (90) such that the magnet (120) and the container (90) held by the holder (110) are close to each other in the approach position (61). With this configuration, the magnet (120) and the container (90) held by the holder (110) can be sufficiently close to each other in the approach position (61), whereby the magnetic particles (82) can be effectively collected.

In the configuration in which the holder exposes the side surface, on the magnet side, of the held container, preferably, the holder (110) has a holding hole (111) for holding the container (90), and an open side surface (113) having, formed therein, an opening that exposes the side surface of the container (90) disposed in the holding hole (111). In the approach position (61), the magnet (120) is disposed so as to be in contact with the open side surface (113). With this configuration, in the approach position (61), the magnet (120) can be brought as close as possible to the container (90) in the holding hole (111). In addition, since positioning of the magnet (120) is achieved when the magnet (120) comes into contact with the open side surface (113), the distance between the magnet (120) and the container (90) in the approach position (61) can be kept constant, and the magnetic collection state during suction on the liquid component by the suction tube (150) can be stabilized without variation.

In the BF separator according to the first aspect, preferably, the approach position (61) and the separate position (62) are positions that allow at least one of the holder (110) and the magnet (120) to be disposed on a straight line in the up-down direction. With this configuration, at least one of the holder (110) and the magnet (120) need not be moved in the horizontal direction, whereby the space, in the horizontal direction, necessary for movement between the approach position (61) and the separate position (62) can be reduced.

In the BF separator according to the first aspect, preferably, the approach position (61) is located below the separate position (62). The suction tube (150) is required to have a certain length in the up-down direction in order to suck the liquid component from the container (90) in the approach position (61), and a relatively large space tends to be required in the up-down direction because the suction tube (150) is connected to a liquid feed tube or the like to transfer the sucked liquid component. Therefore, when the approach position (61) is located on the lower side while the separate position (62) is located on the upper side, a space, in the up-down direction, required for the suction tube (150) to suck the liquid component can be ensured by using the space for moving the holder (110) in the approach position (61) to the upper separate position (62). Thus, downsizing of the BF separator can be achieved by the effective use of the space between the approach position (61) and the separate position (62).

In this case, preferably, the BF separator further includes a washer (153) which is disposed at substantially the same height position as the holder (110) in the approach position (61), and is configured to wash the suction tube (150). The suction tube (150) is configured to move between the container (90) held by the holder (110) in the approach position (61), and the washer (153). With this configuration, since the holder (110) in the approach position (61) and the washer (153) are disposed at substantially the same height, the movement route of the suction tube (150) between the washer (153) and the container (90) in the approach position (61) can be simplified.

In the BF separator according to the first aspect, preferably, the movement mechanism (130) is configured to move the holder (110) not only to the approach position (61) and the separate position (62) but also to a container setting position (63) where setting and removal of the container (90) are performed. For example, when the container (90) is set in or removed from the holder (110) in the approach position (61), an operation to prevent interference of the container (90) being transferred with the suction tube (150) is likely to be required. Meanwhile, when setting and removal of the container (90) in/from the holder (110) are performed in the container setting position (63), interference of the container (90) being transferred with each component in the BF separator (100) can be easily avoided.

In this case, preferably, in the container setting position (63), the holder (110) and the magnet (120) are configured to approach each other over a distance that allows the magnetic particles (82) in the container (90) to be magnetically collected. With this configuration, magnetic collection of the magnetic particles (82) can be started from when the container (90) is set in the holder (110) in the container setting position (63). Therefore, the time required for magnetic collection of the magnetic particles (82) can be reduced, as compared to the case where magnetic collection is performed only in the approach position (61).

In the configuration in which the movement mechanism moves the holder to the container setting position, preferably, the container setting position (63) is located above the approach position (61) and the separate position (62). With this configuration, the suction tube (150) performing suction of the liquid component in the approach position (61) and the agitator (140) performing agitation of the sample in the separate position (62) are less likely to become obstacles when setting/removal of the container (90) is performed in the container setting position (63). Therefore, it is possible to easily avoid interference with the components related to setting/removal of the container (90) in/from the holder (110).

In the BF separator according to the first aspect, preferably, the movement mechanism (130) is configured to move the holder (110), the magnet (120), and the agitator (140) in conjunction with one another. With this configuration, not only the holder (110) and the magnet (120) but also the agitator (140) can be moved in conjunction with one another by the movement mechanism (130). Therefore, the device configuration for causing the agitator (140) to agitate the sample in the container (90) held by the holder (110) in the separate position (62) can be simplified.

In the BF separator according to the first aspect, preferably, the movement mechanism (130) includes a common drive unit (131) configured to move both the holder (110) and the magnet (120). With this configuration, since a drive source can be shared by the holder (110) and the magnet (120), the configuration of the movement mechanism (130) can be simplified. As a result, the installation area of the BF separator can be further reduced.

In this case, preferably, the BF separator further includes a movement base (160) on which the holder (110) and the magnet (120) are provided. The movement mechanism (130) causes the drive unit (131) to move the movement base (160). With this configuration, by only moving the movement base (160), the movement mechanism (130) can move both the holder (110) and the magnet (120) in conjunction with one another. Therefore, the configuration of the movement mechanism (130) can be further simplified.

In the configuration further including the movement base, preferably, the agitator (140) is provided on the movement base (160), and is configured to agitate the sample in the container (90) when the holder (110) is driven. With this configuration, since not only the holder (110) and the magnet (120) but also the agitator (140) can be provided on the movement base (160), downsizing of the BF separator (100) can be effectively achieved. In contrast to the case where, for example, an agitation rod or the like is inserted in the container (90), the agitator (140) only has to vibrate the holder (110). Therefore, the configuration of the agitator (140) is simplified, and the downsizing of the agitator (140) is achieved.

In the configuration further including the movement base, preferably, a plurality of processing ports (101) each including the holder (110) and the magnet (120) are provided on the movement base (160). With this configuration, a plurality of containers (90) can be set on the single movement base (160), and the samples in the plurality of containers (90) can be subjected to the BF separation process. Therefore, the installation area of the BF separator (100) can be effectively reduced.

A second aspect of the present invention relates to a sample analyzer including: a container transfer unit (510) configured to transfer a container (90) which stores therein a sample containing a test substance (81), magnetic particles (82) that bind to the test substance (81), and a labeled substance (83) that binds to the test substance (81); a BF separator (100) according to the first aspect, configured to separate the magnetic particles (82) from a liquid component in the sample in the container (90) transferred by the container transfer unit (510); and a detection unit (520) configured to detect the test substance (81) bound to the magnetic particles (82) in the container (90) from which the liquid component is separated by the BF separator (100).

Since the sample analyzer according to the second aspect is provided with the BF separator (100) according to the first aspect, the BF separation process (magnetic collection, insertion of the suction tube, suction, and agitation) can be performed in a position different from the container setting position. In addition, with the simple configuration, the container (90) and the magnet (20) can be moved to the positional relationship for the agitation. Therefore, the degree of freedom in the device configuration of the BF separator (100) can be increased. As a result, the degree of freedom in the configuration related to the BF separator (100) can be increased in the sample analyzer, whereby the degree of freedom in the device configuration of the sample analyzer as a whole can be increased.

A third aspect of the present invention relates to a BF separation method including: moving a holder (10) holding a container (90) which stores therein a sample containing magnetic particles (82) and a liquid component, and causing a magnet (20) to approach the holder (10) in conjunction with the movement of the holder (10); sucking the liquid component in the container (90) held by the holder (10) located in an approach position (61) where the holder (10) and the magnet (20) are close to each other; discharging a washing liquid into the container (90) from which the liquid component has been sucked; moving the holder (10) from the approach position (61), and causing the magnet (20) to separate from the holder (10) in conjunction with the movement of the holder (10); and agitating the sample in the container (90) held by the holder (10) located in a separate position (62) where the holder (10) and the magnet (20) are away from each other.

In the BF separation method according to the third aspect, the above-described configuration allows the holder (10) and the magnet (20) to move in conjunction with each other. Therefore, by only moving the holder (10) in which the container (90) is set, the holder (10) and the magnet (20) can be moved to the separate position (62) where they are away from each other and to the approach position (61) where they are close to each other. Therefore, the BF separation process (magnetic collection, insertion of the suction tube, suction, and agitation) can be performed in a position different from the container setting position. In addition, with the simple configuration, the container (90) and the magnet (20) can be moved to the positional relationship for the magnetic collection, and the container (90) and the magnet (20) can be moved to the positional relationship for the agitation. Consequently, the degree of freedom in the device configuration can be increased.

### ADVANTAGEOUS EFFECTS OF THE INVENTION

According to the present invention, the degree of freedom in the device configuration can be increased.

### BRIEF DESCRIPTION OF THE DRAWINGS

[FIG. 1] FIG. 1 is a schematic diagram showing an outline of a BF separator according to one embodiment.
[FIG. 2] FIG. 2 is a schematic diagram showing a configuration example of the BF separator including a discharge tube.
[FIG. 3A] FIG. 3A is a schematic diagram illustrating a BF separation method according to the embodiment.
[FIG. 3B] FIG. 3B is a schematic diagram illustrating the BF separation method according to the embodiment.
[FIG. 3C] FIG. 3C is a schematic diagram illustrating the BF separation method according to the embodiment.
[FIG. 4] FIG. 4 is a schematic diagram showing an outline of a sample analyzer according to the embodiment.
[FIG. 5] FIG. 5 is a schematic diagram showing a configuration example of the BF separator.
[FIG. 6A] FIG. 6A shows a first configuration example of a holder and a magnet in a separate position.
[FIG. 6B] FIG. 6B shows the first configuration example of the holder and the magnet in an approach position.
[FIG. 7A] FIG. 7A shows a second configuration example of the holder and the magnet in the separate position.
[FIG. 7B] FIG. 7B shows the second configuration example of the holder and the magnet in the approach position.
[FIG. 8] FIG. 8 shows an example in which the holder also moves to a container setting position.
[FIG. 9] FIG. 9 shows combinations (A) to (F) of positional relationships between the approach position, the separate position, and the container setting position.
[FIG. 10] FIG. 10 shows a configuration example of the BF separator including a guide portion.
[FIG. 11] FIG. 11 is a schematic plan view showing a configuration example of the sample analyzer.
[FIG. 12] FIG. 12 is a vertical cross-sectional view of the BF separator in the approach position.
[FIG. 13] FIG. 13 is a vertical cross-sectional view of the BF separator in the separate position.
[FIG. 14] FIG. 14 is a vertical cross-sectional view of the BF separator in the container setting position.
[FIG. 15A] FIG. 15A is a schematic diagram showing the positions of the guide portion and a rotating body in the approach position.
[FIG. 15B] FIG. 15B is a schematic diagram showing the positions of the guide portion and the rotating body in the container setting position.
[FIG. 15C] FIG. 15C is a schematic diagram showing the positions of the guide portion and the rotating body in the separate position.
[FIG. 16] FIG. 16 is a vertical cross-sectional view showing a configuration example of a movement base.
[FIG. 17] FIG. 17 is a top view of the BF separator shown in FIG. 12.
[FIG. 18] FIG. 18 is a flowchart showing the flow of a BF separation process performed by the BF separator shown in FIG. 12.
[FIG. 19] FIG. 19 is a schematic diagram illustrating the content of the BF separation process shown in FIG. 18.
[FIG. 20] FIG. 20 illustrates an analysis process performed by the sample analyzer.
[FIG. 21] FIG. 21 is a flowchart illustrating the analysis process shown in FIG. 20.

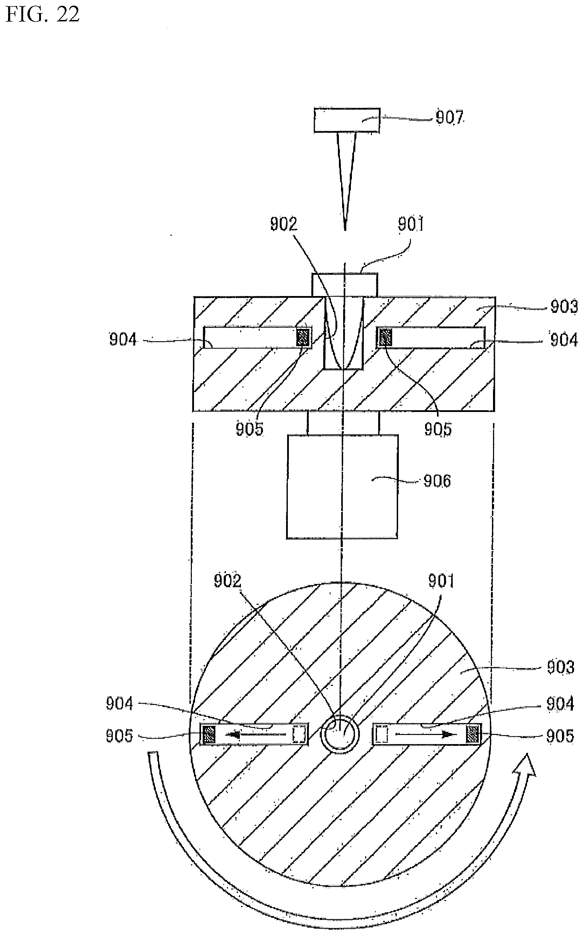

[FIG. 22] FIG. 22 is a diagram showing the conventional art.

### DESCRIPTION OF EMBODIMENTS

Hereinafter, embodiments will be described with reference to the drawings.

### [Outline of BF separator]

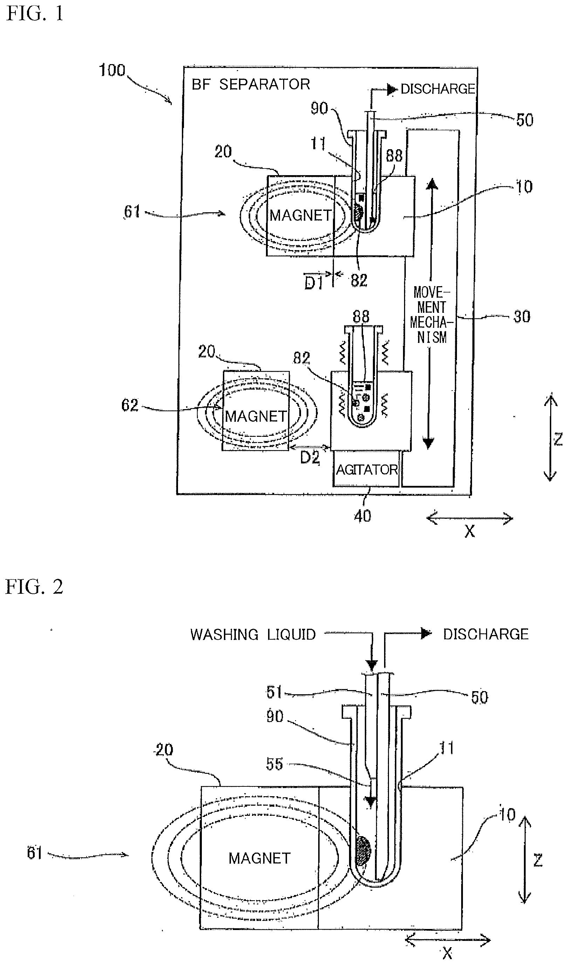

First, the outline of a BF separator 100 according to one embodiment will be described with reference to FIG. 1.

The BF separator 100 is a device for performing a BF separation process of separating a solid phase (bound) having a test substance bound thereto from a liquid component containing a substance (free), other than the solid phase, having the test substance bound thereto.

The test substance may contain, for example, a predetermined component, a cell, or a solid component in a blood specimen or a urine specimen. The test substance may be nucleic acid such as DNA (deoxyribonucleic acid), a cell and an intracellular substance, an antigen or antibody, a protein, a peptide, or the like.

The BF separation process is executed in an automated sample analyzer for analyzing a test substance as an analysis target in a liquid sample. In the sample analyzer, a complex in which the test substance, the solid phase, and a labeled substance bind to each other is formed, and detection of the test substance is automatically performed based on the label. The sample analyzer is, for example, an immunoassay apparatus, and binds the test substance, the solid phase, and the labeled substance to each other by using antigen-antibody reaction. The immunoassay apparatus detects, as the test substance, an antigen or an antibody, a protein, a peptide, or the like that is contained in blood. The immunoassay apparatus obtains serum or plasma as a specimen, and quantitatively or qualitatively measures an antigen, an antibody, or the like contained in the specimen. The antigen-antibody reaction includes not only reaction between an antigen and an antibody but also reaction using a specifically binding substance such as an aptamer. The aptamer is a nucleic acid molecule or a peptide synthesized so as to specifically bind to a specific substance.

In the BF separation process, magnetic particles 82 are used as the solid phase in order to efficiently perform separation of the solid phase and the liquid component. When the magnetic particles 82 are used, a liquid component 88 other than the magnetic particles 82 is sucked while the magnetic particles 82 having the test substance bound thereto are collected by a magnetic force, whereby the magnetic particles 82 and the liquid component 88 are separated from each other. The magnetic particle 82 is a particle containing a magnetic body, and the surface of the magnetic particle 82 is coated with a component for binding to the test substance or the like. The magnetic particle 82 may be any particle that contains a magnetic material as a base and is used for conventional immunoassay. For example, a magnetic particle containing, as the base, Fe₂O₃ and/or Fe₃O₄, cobalt, nickel, ferrite, magnetite, or the like, can be used.

The magnetic particle 82 directly or indirectly binds to the test substance. That is, the magnetic particle 82 and the test substance may directly bind to each other through antigen-antibody reaction. When the test substance is an antigen or an antibody, the magnetic particle 82 can be a magnetic particle having immobilized thereon an antibody or an antigen that specifically binds to the antigen or the antibody.

The magnetic particle 82 and the test substance may bind to each other via a capture substance that binds to the magnetic particle 82 and to the test substance. The capture substance contains a binding substance that binds to the magnetic particle 82. The magnetic particle 82 has, immobilized thereon, a substance that specifically binds to the capture substance. For binding between the binding substance and a solid phase carrier, a combination such as: biotin and avidins; hapten and anti-hapten antibody; nickel and histidine tag; or glutathione and glutathione-S-transferase, can be used, for example. Here, "avidins" means that both avidin and streptavidin are included. The capture substance contains an antibody or an antigen that specifically binds to the antigen or the antibody as the test substance. Thus, the test substance and the capture substance bind to each other, and the capture substance and the magnetic particle 82 bind to each other.

As shown in FIG. 1, the BF separator 100 according to the present embodiment includes, as main components, a holder 10 for holding a container 90 which stores a sample therein, a magnet 20, a movement mechanism 30, an agitator 40, and a suction tube 50. In FIG. 1, an up-down direction as a vertical direction is defined as a Z direction, and one of horizontal directions orthogonal to the up-down direction is defined as an X direction.

The container 90 is, for example, a reaction container that has a cylindrical shape having an opening at one end and a closed bottom at the other end, and that can store therein a liquid such as a specimen or a reagent. The container 90 is, for example, a disposable container made of resin. In this case, a reaction container that has been used can be discarded as it is. In the container 90, the sample containing the magnetic particles 82 and the liquid component 88 is stored.

The holder 10 can hold the container 90 storing the sample containing the magnetic particles 82 and the liquid component 88. The holder 10 holds the container 90 such that the opening of the container 90 faces upward and the container 90 does not move to an extent equal to or more than a prescribed range. In the configuration example shown in FIG. 1, the holder 10 is a structure having, formed therein, a holding hole 11 in which the container 90 can be inserted. The holding hole 11 is opened at the upper surface of the holder 10, and therefore can hold the container 90 inserted therein from above. The holder 10 may have, for example, a hand-like structure that grips and holds the container 90 at the side surface of the container 90, or an engagement structure that is engaged with a part of the container 90, such as a flange-like portion formed at an upper end of the container 90, and holds the container 90 in a suspended manner.

The magnet 20 can magnetically collect magnetic particles. That is, the magnet 20 causes a magnetic force to act on the container 90 held by the holder 10, thereby collecting the magnetic particles 82 in the container 90. The magnet 20 is a permanent magnet, for example.

The magnet 20 is movable relative to the holder 10 in a direction approaching or separating from the holder 10. That is, one or both of the magnet 20 and the holder 10 are movable in a direction approaching each other or separating from each other. In the configuration example shown in FIG. 1, the magnet 20 is movable in a direction approaching the holder 10 and in a direction separating from the holder 10. The direction of the relative movement between the magnet 20 and the holder 10 is not particularly limited. In FIG. 1, the magnet 20 is movable relative to the holder 10 in the horizontal direction approximately orthogonal to the up-down direction. When the magnet 20 approaches the holder 10, the magnetic particles 82 in the container 90 are collected to the magnet 20 side by the magnetic force of the magnet 20. When the magnet 20 is away from the holder 10, the magnetic force does not substantially act on the magnetic particles 82, or the magnetic force acting on the magnetic particles 82 is weakened such that the magnetic particles 82 are dispersed by agitation. Therefore, when the magnet 20 is away from the holder 10, magnetic collection of the magnetic particles 82 is canceled.

The movement mechanism 30 can move both the holder 10 and the magnet 20 in conjunction with one another. The movement mechanism 30 moves the holder 10, and moves the magnet 20 in conjunction with the movement of the holder 10, in a direction in which the holder 10 and the magnet 20 approach each other or separate from each other. The movement mechanism 30 can move both the holder 10 and the magnet 20 in conjunction with one another to at least an approach position 61 and a separate position 62. The approach position 61 and the separate position 62 may be positions at different heights, or may be positions at the same height and apart from each other in the horizontal direction. When the approach position 61 and the separate position 62 are at different heights, it does not matter which one of the position 61 and the position 62 is above the other. The movement mechanism 30 moves both the holder 10 and the magnet 20 so that the height is changed in at least the up-down direction.

In FIG. 1, the approach position 61 and the separate position 62 are positions at different heights. The movement mechanism 30 moves both the holder 10 and the magnet 20 in the up-down direction. This enables the magnetic collection process in the approach position 61 and the later-described agitation process in the separate position 62 to be performed at different positions in the up-down direction. Therefore, as compared to the case where the approach position 61 and the separate position 62 are arranged in a horizontal plane, the movement distance of the holder 10 in the horizontal direction between the approach position 61 and the separate position 62 can be reduced by the difference in height, and thus the installation area of the BF separator 100 in the horizontal direction can be reduced. Note that FIG. 1 indicates that one holder 10 and one magnet 20 move to the approach position 61 and to the separate position 62, and does not indicate that two holders 10 and two magnets 20 are provided.

The movement mechanism 30 consists of one or a plurality of direct acting mechanisms. The direct acting mechanism consists of a linear motor, a combination of a motor and a linear guide, or the like. The movement mechanism 30 may be configured to rotate in the up-down direction around a rotation shaft in the horizontal direction. The movement mechanism 30 includes at least one drive source such as a motor. For example, the movement mechanism 30 causes separate drive sources to move the holder 10 and the magnet 20 in conjunction with one another. Alternatively, for example, the movement mechanism 30 causes a common drive source to move the holder 10 and the magnet 20.

In the approach position 61, the relative movement of the holder 10 and the magnet 20 allows the holder 10 and the magnet 20 to approach each other. In the separate position 62, the relative movement of the holder 10 and the magnet 20 allows the holder 10 and the magnet 20 to separate from each other more than in the approach position 61. That is, in the approach position 61, the distance between the holder 10 and the magnet 20 is D1, while, in the separate position 62, the distance between the holder 10 and the magnet 20 is D2 greater than the distance D1. As a result, in the approach position 61, the magnetic particles 82 in the container 90 held by the holder 10 are magnetically collected. As a result, in the separate position 62, magnetic collection of the magnetic particles 82 in the container 90 held by the holder 10 is canceled. For example, a drive source different from the movement mechanism 30 causes the holder 10 and the magnet 20 to relatively move in a direction in which the holder 10 and the magnet 20 approach each other or separate from each other. Alternatively, as described later, for example, the holder 10 and the magnet 20 are guided when being moved by the movement mechanism 30, and relatively move in a direction in which the holder 10 and the magnet 20 approach each other or separate from each other, without using a different drive source.

The agitator 40 can agitate the sample in the container 90. The agitator 40 can cause the sample in the container 90, which is held by the holder 10 in the separate position 62 where the holder 10 and the magnet 20 are away from each other, to move in the container 90, thereby agitating the sample. The agitating method by the agitator 40 is not particularly limited. For example, the agitator 40 vibrates the container 90 held by the holder 10 to agitate the sample in the container 90. For example, the agitator 40 vibrates the holder 10 holding the container 90 to agitate the sample. Such vibration may be caused by using a vibration motor or by rotating the container 90 or the holder 10 with a rotary motor, for example. Alternatively, for example, the agitator 40 inserts an agitation rod from the opening of the container 90, and agitates the sample with the agitation rod. In this case, the agitator 40 may be configured to be movable so that the agitation rod can be put in and out. In the configuration example shown in FIG. 1, the agitator 40 vibrates the holder 10 to agitate the sample in the container 90.

The agitator 40 agitates the sample in the container 90 held by the holder 10 in the separate position 62. That is, the agitator 40 agitates the liquid component 88 in the container 90 in a state where the magnet 20 is away from the holder 10 in the separate position 62 and therefore magnetic collection of the magnetic particles 82 in the container 90 held by the holder 10 is canceled.

In the container 90, an unnecessary substance is present together with the magnetic particles 82 bound to the test substance. The unnecessary substance does not contribute to detection of the test substance, or becomes a noise source or the like. Examples of the unnecessary substance include: a component, other than the test substance, contained in the specimen; a labeled substance and a capture substance that have not bound to the magnetic particles 82; and other unspecified impurities. As a result of the agitation, not only the magnetic particles 82 but also the unnecessary substance in the container 90 can be widely dispersed in the liquid component 88.

The suction tube 50 can suck the liquid component 88 in the container 90. The suction tube 50 enters the container 90 held by the holder 10 in the approach position 61 where the holder 10 and the magnet 20 are close to each other, and sucks the liquid component 88 in the container 90. The suction tube 50 is connected to a fluid circuit via a liquid feed path (not shown), and sucks a liquid from a tip thereof. In the present embodiment, the suction tube 50 sucks the liquid component 88 in the container 90 held by the holder 10 in the approach position 61. For example, the suction tube 50 is configured to be movable such that the suction tube 50 can enter and withdraw from the container 90 held by the holder 10. For example, the suction tube 50 is disposed at a position aligned with the holder 10 disposed in the approach position 61, and enters the container 90 when the container 90 held by the holder 10 is moved to the approach position 61 by the movement mechanism 30.

The suction tube 50 sucks the liquid component 88 in the container 90 in a state where the magnet 20 approaches the holder 10 in the approach position 61 and therefore the magnetic particles 82 in the container 90 held by the holder 10 are magnetically collected. As a result of the suction, the unnecessary substance contained in the liquid component 88 is discharged to the outside of the container 90 together with the liquid component 88 while the magnetic particles 82 are left in the container 90. Thus, the magnetic particles 82 having the test substance bound thereto are separated from the liquid component 88 containing the substance other than the magnetic particles 82 having the test substance bound thereto.

As described above, in the configuration example shown in FIG. 1, agitation of the sample by the agitator 40 and suction of the liquid component 88 by the suction tube 50 are performed at different height positions. Furthermore, in the configuration example shown in FIG. 1, agitation of the sample by the agitator 40 and suction of the liquid component 88 by the suction tube 50 are performed with the container 90 being held by the holder 10. During the suction of the liquid component 88 in the approach position 61 and the agitation of the sample in the separate position 62, the container 90 is not removed from the holder 10, that is, the holder 10 continues to hold the container 90.

With the above configuration, the BF separator 100 according to the present embodiment includes the movement mechanism 30 which, in conjunction with movement of the holder 10, moves the magnet 20 in the direction in which the holder 10 and the magnet 20 approach each other or separate from each other. Therefore, by only moving the holder 10 in which the container 90 is set, the holder 10 and the magnet 20 can be moved to the separate position 62 where they are away from each other and to the approach position 61 where they are close to each other. Therefore, the BF separation process (magnetic collection, insertion of the suction tube, suction, and agitation) can be performed in a position different from the container setting position. In addition, with the simple configuration, the container 90 and the magnet 20 can be moved to the positional relationship for the magnetic collection, and the container 90 and the magnet 20 can be moved to the positional relationship for the agitation. Consequently, the degree of freedom in the device configuration can be increased.

In the configuration example shown in FIG. 2, the BF separator 100 is provided with a discharge tube 51 which discharges a washing liquid 55 into the container 90 held by the holder 10 in the approach position 61. The discharge tube 51 can discharge the washing liquid 55 into the container 90. The discharge tube 51 enters the container 90 held by the holder 10, and discharges the washing liquid 55 into the container 90. The discharge tube 51 is connected to a washing liquid storage portion via a liquid feed path (not shown) and a fluid circuit (not shown), and therefore can discharge the washing liquid 55 from a tip thereof. For example, the discharge tube 51 is configured to be movable so that the discharge tube 51 can enter and withdraw from the container 90 held by the holder 10. The discharge tube 51 may be configured to enter the container 90 when the container 90 held by the holder 10 is moved to the approach position 61 by the movement mechanism 30.

The discharge tube 51 discharges the washing liquid 55 into the container 90 from which the liquid component 88 has been sucked by the suction tube 50, in a state where the magnet 20 approaches the holder 10 in the approach position 61 and therefore the magnetic particles 82 in the container 90 held by the holder 10 are magnetically collected. The agitator 40 agitates the sample, in the container 90, containing the magnetic particles 82 and the washing liquid 55. As a result, the unnecessary substance nonspecifically attached to the magnetic particles 82 in the container 90 is dispersed in the washing liquid 55. That is, the magnetic particles 82 having the test substance bound thereto are washed by the washing liquid 55.

Thus, both suction of the liquid component 88 by the suction tube 50 and discharge of the washing liquid 55 by the discharge tube 51 can be performed in the approach position 61, and the device configuration can be simplified as compared to the case where suction of the liquid component 88 and discharge of the washing liquid 55 are performed in separate positions.

### [BF separation method]

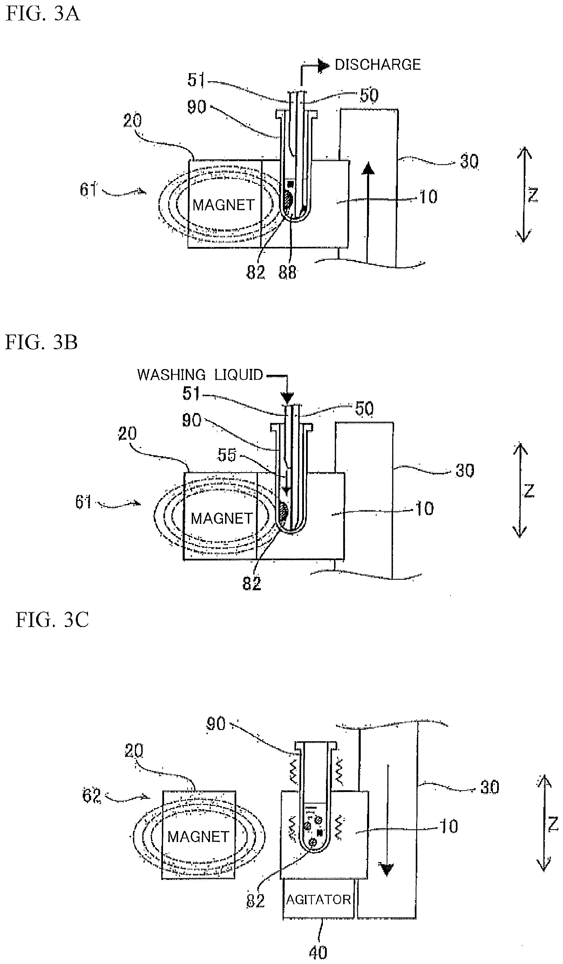

Next, the BF separation method according to the present embodiment will be described with reference to FIG. 3A to FIG. 3C. The BF separation method according to the present embodiment is a method of performing a BF separation process by a BF separator, and includes the following steps. That is, the BF separation method according to the present embodiment includes:
(1) moving the holder 10 holding the container 90 which stores therein the sample containing the magnetic particles 82 and the liquid component 88, and, in conjunction with this movement, causing the magnet 20 to approach the holder 10 (see FIG. 3A);
(2) sucking the liquid component 88 in the container 90 held by the holder 10 in the approach position 61 where the holder 10 and the magnet 20 are close to each other (see FIG. 3A);
(3) discharging the washing liquid 55 into the container 90 from which the liquid component 88 has been sucked (see FIG. 3B);
(4) moving the holder 10 from the approach position 61, and, in conjunction with this movement, causing the holder 10 to separate from the magnet 20 (see FIG. 3C); and
(5) agitating the sample in the container 90 held by the holder 10 in the separate position 62 where the holder 10 and the magnet 20 are away from each other (see FIG. 3C).

Movement and approaching in step (1) are executed in advance of suction of the liquid component 88 in step (2). Suction of the liquid component 88 in step (2) is performed in advance of discharge of the washing liquid 55 in step (3). Movement and separation in step (4) are performed in advance of sample agitation in step (5). Suction of the liquid component 88 in step (2), discharge of the washing liquid 55 in step (3), and sample agitation in step (5) are executed in this order, whereby the magnetic particles 82 and the remaining unnecessary substance can be dispersed in the washing liquid 55 after the liquid component 88 has been discharged from the container 90 in step (2) while the magnetic particles 82 having the test substance bound thereto are left in the container 90. After sample agitation in step (5), suction of the liquid component 88 in step (2) is executed again, whereby the unnecessary substance dispersed in the washing liquid 55 can be discharged together with the washing liquid 55 while the magnetic particles 82 are left. Suction of the liquid component 88 in step (2), discharge of the washing liquid 55 in step (3), and sample agitation in step (5) may be repeated a plurality of number of times. In this case, the BF separation process can be performed more effectively.

In the BF separation method according to the present embodiment, the holder 10 and the magnet 20 are moved in conjunction with one another by the above-described configuration. Therefore, by only moving the holder 10 in which the container 90 is set, the holder 10 and the magnet 20 can be moved to the separate position 62 where they are away from each other and to the approach position 61 where they are close to each other. Therefore, the BF separation process (magnetic collection, insertion of the suction tube, suction, and agitation) can be performed in a position different from the container setting position. In addition, with the simple configuration, the container 90 and the magnet 20 can be moved to the positional relationship for the magnetic collection, and the container 90 and the magnet 20 can be moved to the positional relationship for the agitation. Consequently, the degree of freedom in the device configuration can be increased.

### [Outline of sample analyzer]

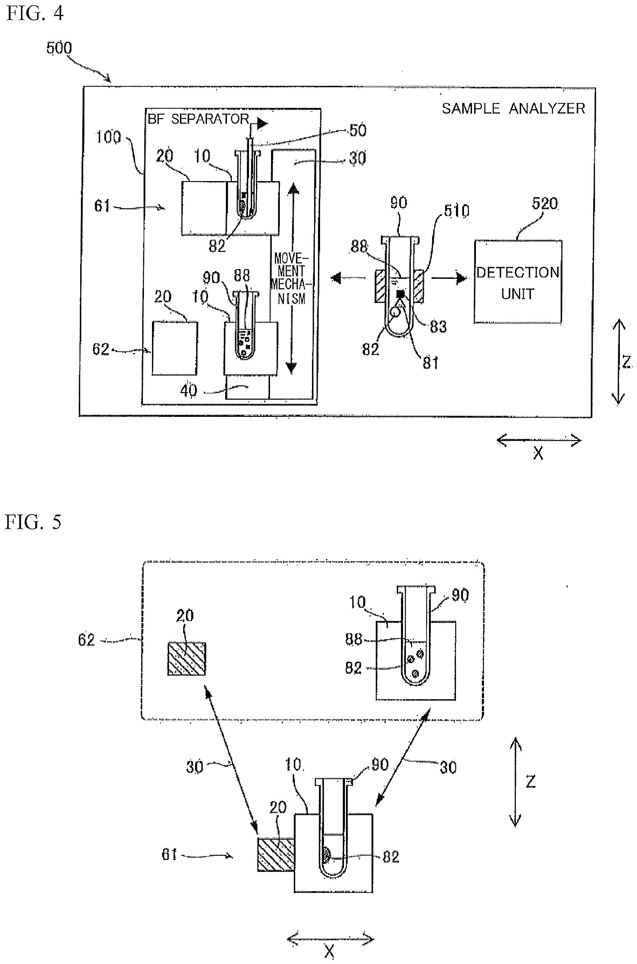

Next, an outline of a sample analyzer 500 according to one embodiment will be described with reference to FIG. 4.

The sample analyzer 500 is a device for analyzing a measurement sample prepared by adding a predetermined reagent to a specimen collected from a subject.

The subject is mostly a human subject, but may be an animal other than a human subject. The sample analyzer 500 performs analysis for a clinical laboratory test or medical research on a specimen collected from a patient, for example. The specimen is derived from an organism. The specimen derived from an organism is, for example, blood (whole blood, serum, or plasma) collected from the subject, or a liquid obtained by subjecting the blood to a predetermined pretreatment. The sample analyzer 500 is an immunoassay apparatus, for example.

The sample analyzer 500 performs the BF separation process on a sample containing a test substance 81, magnetic particles 82 that bind to the test substance 81, and a labeled substance 83 that binds to the test substance 81, thereby separating a substance other than a complex including the test substance 81, the magnetic particles 82, and the labeled substance 83 binding to each other. Thereafter, the sample analyzer 500 detects the test substance 81 on the basis of the label in the labeled substance 83. For example, the sample analyzer 500 adds predetermined one or more kinds of reagents to the specimen to prepare a measurement sample containing the test substance 81, the magnetic particles 82, and the labeled substance 83. In addition, for example, the sample analyzer 500 obtains a sample prepared in advance, and analyzes the obtained sample.

As shown in FIG. 4, the sample analyzer 500 includes a container transfer unit 510, the BF separator 100 shown in FIG. 1, and a detection unit 520.

The container transfer unit 510 can transfer the container 90 which stores therein the sample containing the test substance 81, the magnetic particles 82 that bind to the test substance 81, and the labeled substance 83 that binds to the test substance 81. The container transfer unit 510 can transfer the container 90 to the BF separator 100 and to the detection unit 520. The container transfer unit 510 includes, for example, a grip part which grips the container 90, and a movement mechanism for moving the grip part. The grip part may have, for example, a hand-like structure that grips and holds the container 90 at the side surface of the container 90, or an engagement structure that is engaged with a part of the container 90, such as a flange-like portion formed at an upper end of the container 90, and holds the container 90 in a suspended manner. As another example, the container transfer unit 510 is configured to move a structure having, formed therein, a holding hole 11 in which the container 90 can be inserted. As still another example, the container transfer unit 510 has a conveyer structure for transferring a plurality of containers 90 along a predetermined track.

The BF separator 100 obtains the container 90 storing the sample therein, from the container transfer unit 510. That is, the container 90 is set in the holder 10 by the container transfer unit 510. The BF separator 100 performs the above-described BF separation process on the sample in the container 90 held by the holder 10. The BF separator 100 may include the discharge tube 51 shown in FIG. 2.

The detection unit 520 can detect the test substance 81 bound to the magnetic particles 82 in the container 90. The detection unit 520 receives, from the container transfer unit 510, the container 90 storing therein the sample that has been subjected to the BF separation process by the BF separator 100. The detection unit 520 detects the test substance 81 bound to the magnetic particles 82 in the container 90 in which the liquid component 88 was separated by the BF separator 100. For example, the detection unit 520 detects a signal based on the label included in the complex in the container 90. Based on the detection result of the detection unit 520, the sample analyzer 500 analyzes, for example, presence/absence of the test substance 81, the number or quantity of the test substance 81, and the concentration or abundance of the test substance 81. The detection may be performed by an appropriate method according to the kind of the label used for the labeled substance 83, and the detection method is not limited in particular.

The labeled substance 83 contains a label that binds to the test substance 81 through an antigen-antibody reaction, and that is detectable by the detection unit 520. The labeled substance 83 is not limited in particular as long as it is an antibody including a well-known label. In a case where a capture substance is used, the labeled substance 83 may bind to the capture substance. Examples of the label contained in the labeled substance include an enzyme, a fluorescent substance, and a radioisotope. Examples of the enzyme include alkaline phosphatase (ALP), peroxidase, glucose oxidase, tyrosinase, and acid phosphatase. As the fluorescent substance, fluorescein isothiocyanate (FITC), green fluorescent protein (GFP), luciferin, or the like can be used. As the radioisotope, 1251, 14C, 32P, or the like can be used. In the example shown in FIG. 4, the label used for the labeled substance 83 is an enzyme.

When the label is an enzyme, a substrate for the enzyme of the labeled substance 83 may be appropriately selected from well-known substrates in accordance with the enzyme. For example, when the enzyme is alkaline phosphatase, examples of the substrate include: chemiluminescent substrates such as CDP-Star (registered-trademark), (disodium 4-chloro-3-(methoxyspiro{1,2-dioxetane-3,2'-(5'-chloro)tricyclo[3.3.1.13,7]decan}-4-yl)phenylphosphate), and CSPD (registered-trademark) (disodium 3-(4-methoxyspiro{1,2-dioxetane-3,2-(5'-chloro)tricyclo[3.3.1.13,7]decane}-4-yl)phenylphosphate); luminescent substrates such as p-nitrophenyl phosphate, 5-bromo-4-chloro-3-indolyl phosphate (BCIP), 4-nitro blue tetrazolium chloride (NBT), and iodonitrotetrazolium (INT); fluorescent substrates such as 4-methylumbelliferyl phosphate (4MUP); chromogenic substrates such as 5-bromo-4-chloro-3-indolyl phosphate (BCIP), disodium 5-bromo-6-chloro-indolyl phosphate, and p-nitrophenyl phosphate; and the like.

For example, when the label used for the labeled substance 83 is an enzyme, the detection unit 520 measures the light, color, or the like that is generated as a result of causing a substrate to react with the enzyme. As the detection unit 520 in this case, a spectrophotometer, a luminometer, or the like can be used. When the labeled substance is a radioisotope, a scintillation counter or the like can be used as the detection unit 520.

In the above configuration, since the sample analyzer 500 according to the present embodiment is provided with the above-described BF separator 100, the BF separation process (magnetic collection, insertion of the suction tube, suction, and agitation) can be performed in a position different from the container setting position. In addition, with the simple configuration, the container 90 and the magnet 20 can be moved to the positional relationship for the agitation. Therefore, the degree of freedom in the device configuration of the BF separator 100 can be increased. As a result, the degree of freedom in the configuration related to the BF separator 100 can be increased in the sample analyzer, whereby the degree of freedom in the device configuration of the sample analyzer as a whole can be increased.

### [Configuration example of BF separator]

In a configuration example shown in FIG. 5, the movement mechanism 30 moves both the holder 10 and the magnet 20 in conjunction with one another in diagonal directions. The movement mechanism 30 need not linearly move the holder 10 and the magnet 20 in the up-down direction as shown in FIG. 1. In the configuration example shown in FIG. 5, the movement mechanism 30 moves the holder 10 and the magnet 20 in conjunction with one another in different directions. In the configuration example shown in FIG. 5, the movement mechanism 30 moves both the holder 10 and the magnet 20 downward to the approach position such that the holder 10 and the magnet 20 approach each other, and moves both the holder 10 and the magnet 20 upward to the separate position 62 such that the holder 10 and the magnet 20 separate away from each other. In the configuration example shown in FIG. 5, the movement mechanism 30 may include a first movement mechanism for moving the holder 10 and a second movement mechanism for moving the magnet 20.

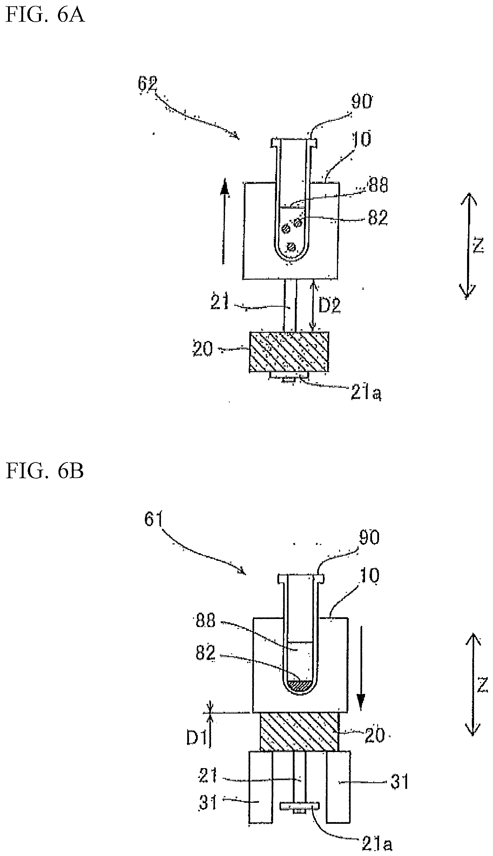

In a configuration example shown in FIG. 6A and FIG. 6B, the holder 10 and the magnet 20 are coupled to each other so as to be relatively movable via a coupling member 21. The magnet 20 is disposed on the lower surface side of the holder 10, and the coupling member 21 extending downward from the lower surface of the holder 10 penetrates through the magnet 20. Between the lower surface of the holder 10 and a pull-out stop portion 21a fixed to a lower end portion of the coupling member 21, the magnet 20 is movable relative to the holder 10 in a direction approaching or separating from the holder 10. As shown in FIG. 6A, the movement mechanism 30 raises the holder 10 to move the holder 10 to the separate position 62 on the upper side. In the separate position 62, the magnet 20 is separated, by its own weight, from the holder 10 at a distance D2. As a result, in the separate position 62, magnetic collection by the magnet 20 is canceled.

As shown in FIG. 6B, the movement mechanism 30 lowers the holder 10 to move the holder 10 to the approach position 61 on the lower side. In the approach position 61, a guide portion 31 is disposed. The guide portion 31 supports the magnet 20 from the lower side by coming into contact with the magnet 20 being lowered together with the holder 10 by the movement mechanism 30. When reaching the approach position 61, the magnet 20 comes into contact with the guide portion 31 which restricts the movement of the magnet 20, whereby the magnet 20 relatively moves in a direction approaching the holder 10 until the distance therebetween becomes D1 (<D2). As a result, in the approach position 61, the magnet 20 causes the magnetic particles 82 in the container 90 to be magnetically collected on the bottom surface side of the container 90.

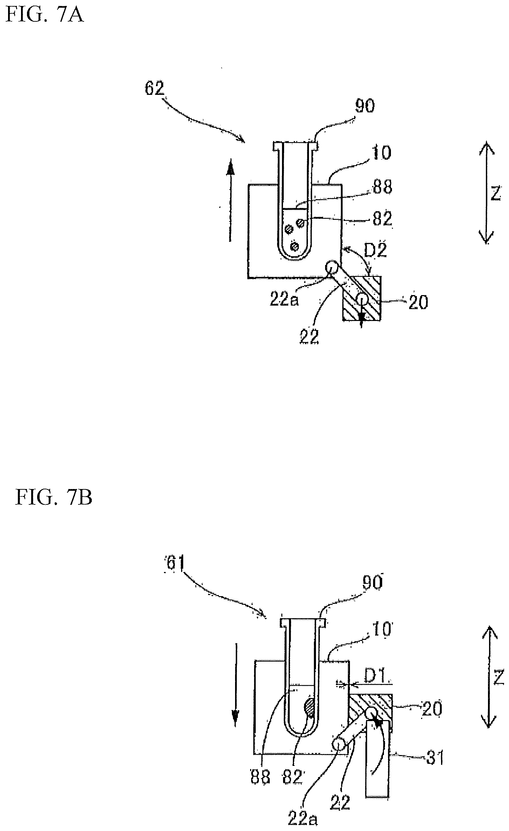

In a configuration example shown in FIG. 7A and FIG. 7B, the holder 10 and the magnet 20 relatively move along a nonlinear track. The magnet 20 is disposed laterally to the side surface of the holder 10, and is held rotatably around a rotation center 22a via a coupling member 22 disposed at a corner portion of the holder 10. As shown in FIG. 7A, when the holder 10 is raised to the separate position 62, the coupling member 22, by using a rotation stop (not shown), holds the magnet 20 at a position the distance D2 away from the holder 10. As shown in FIG. 7B, when the holder 10 reaches the approach position 61 on the lower side, the magnet 20 comes into contact with the guide portion 31, rotates around the rotation center 22a with respect to the holder 10, and relatively moves in a direction approaching the holder 10 until the distance therebetween becomes D1 (<D2). As a result, magnetic collection by the magnet 20 is canceled in the separate position 62, and the magnetic particles 82 are magnetically collected on the side surface of the container 90 by the magnet 20 in the approach position 61.

In the configuration examples shown in FIG. 6A, FIG. 6B, FIG. 7A, and FIG. 7B, the movement mechanism 30 is connected to the holder 10 and moves the holder 10. Since the holder 10 and the magnet 20 are coupled to each other, both the holder 10 and the magnet 20 are moved in conjunction with one another.

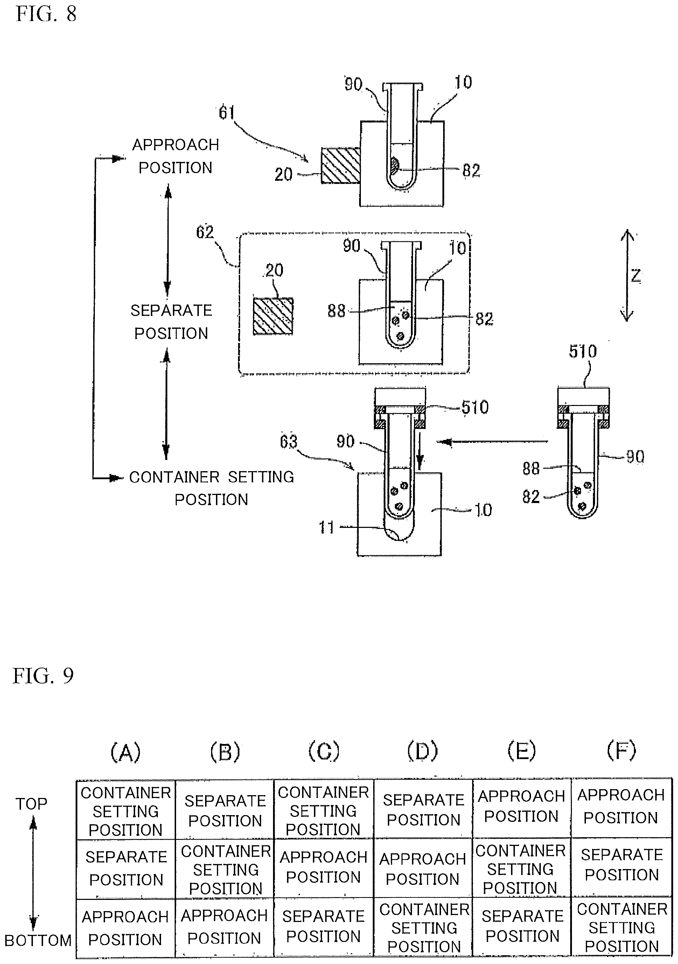

In a configuration example shown in FIG. 8, the movement mechanism 30 moves the holder 10 and the magnet 20, in conjunction with one another, not only to the approach position 61 and the separate position 62 but also to a container setting position 63. In FIG. 8, the container setting position 63 is located at a height different from the heights of the approach position 61 and the separate position 62.

Specifically, the movement mechanism 30 is configured to move the holder 10 not only to the approach position 61 and the separate position 62 but also to the container setting position 63 where setting and removal of the container 90 are performed. That is, the container transfer unit 510 performs setting and removal of the container 90 with respect to the holder 10 disposed in the container setting position 63. For example, when the container 90 is set in and removed from the holder 10 in the approach position 61, an operation to prevent interference of the suction tube 50 with the container 90 being transferred is likely to be required. Meanwhile, when the container 90 is set in and removed from the holder 10 in the container setting position 63, interference of the container 90 being transferred with each component in the BF separator 100 can be easily avoided.

In the configuration example shown in FIG. 8, the approach position 61 is located above the separate position 62. The container setting position 63 is located beneath the approach position 61 and the separate position 62. That is, the approach position 61, the separate position 62, and the container setting position 63 are arranged in this order from the top.

The order of the approach position 61, the separate position 62, and the container setting position 63 in the height direction is not limited to that shown in FIG. 8. FIG. 9 shows combinations (A) to (F) of the approach position 61, the separate position 62, and the container setting position 63 according to their heights. In FIG. 9, the upper side indicates the higher position while the lower side indicates the lower position. For example, in FIG. 9(A), the container setting position 63, the separate position 62, and the approach position 61 are arranged in this order from the top. The order of the approach position 61, the separate position 62, and the container setting position 63 in the height direction may be any of the combinations (A) to (F) shown in FIG. 9. The movement mechanism 30 may move the holder 10 and the magnet 20 to a fourth position and a fifth position at heights different from the heights of the approach position 61, the separate position 62, and the container setting position 63.

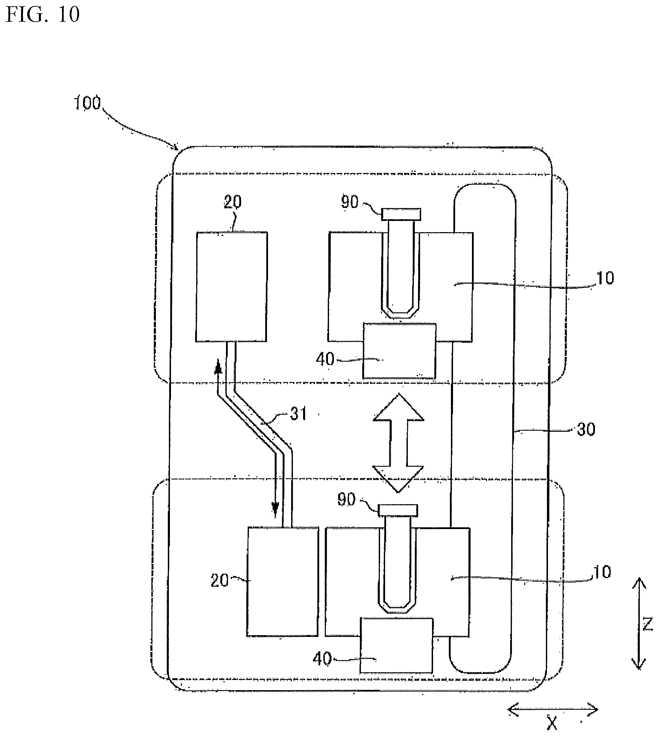

In a configuration example shown in FIG. 10, the movement mechanism 30 is provided so as to linearly extend in the up-down direction, and moves both the holder 10 and the magnet 20 in the up-down direction. The movement mechanism 30 moves both the holder 10 and the magnet 20 to the approach position 61 on the lower side and to the separate position 62 on the upper side with respect to the approach position 61. The holder 10 and the magnet 20 are movable in the up-down direction by the movement mechanism 30. The holder 10 and the magnet 20 can relatively move so as to approach each other or separate from each other in a direction intersecting the up-down direction in which they are moved by the movement mechanism 30. In the example shown in FIG. 10, the magnet 20 can move relative to the holder 10 in the horizontal direction intersecting the up-down direction.

As described above, in the configuration example shown in FIG. 10, the movement mechanism 30 is configured to move the holder 10 and the magnet 20 in the up-down direction, and the holder 10 and the magnet 20 relatively move so as to approach each other or separate from each other in the direction intersecting the up-down direction, in conjunction with the movement in the up-down direction. Thus, the movement mechanism 30 only has to simply move the holder 10 and the magnet 20 in the up-down direction, and need not move them in the horizontal direction. Therefore, the configuration of the movement mechanism 30 can be simplified, and the installation area of the movement mechanism 30 can be reduced. As a result, the installation area of the BF separator 100 can be further reduced.

In the configuration example shown in FIG. 10, the BF separator 100 includes the guide portion 31 which comes into contact with at least one of the holder 10 and the magnet 20 which are moved by the movement mechanism 30, and causes the holder 10 and the magnet 20 to approach each other or separate from each other. Thus, using movements of the holder 10 and the magnet 20 to the approach position 61 and to the separate position 62 by the movement mechanism 30, the holder 10 and the magnet 20 can be caused to approach each other or separate from each other by the contact with the guide portion 31 during the movement. Therefore, it is not necessary to provide a drive source for relatively moving the holder 10 and the magnet 20, in addition to the movement mechanism 30 for moving them between the approach position 61 and the separate position 62. Thus, the device configuration can be simplified.

Specifically, in the configuration example shown in FIG. 10, the guide portion 31 comes into contact with the magnet 20, out of the holder 10 and the magnet 20, and guides the movement of the magnet 20. The guide portion 31 extends in the up-down direction along the direction of movement caused by the movement mechanism 30. The position of the guide portion 31 in the horizontal direction changes such that the guide portion 31 approaches the holder 10 in the horizontal direction intersecting the up-down direction as it approaches the approach position 61, and is moved away from the holder 10 in the horizontal direction as it approaches the separate position 62. Thus, when the magnet 20 is moved in the up-down direction by the movement mechanism 30, the guide portion 31 moves the magnet 20 toward the holder 10 as it approaches the approach position 61, and moves the magnet 20 away from the holder 10 as it approaches the separate position 62. Using the driving force, of the movement mechanism 30, that moves the holder 10 and the magnet 20 between the approach position 61 and the separate position 62, the guide portion 31 moves at least one of the holder 10 and the magnet 20 in a direction different from the direction of movement caused by the movement mechanism 30.

The guide portion 31 has a structure, such as a rail or a guide groove, which is engaged with at least one of the holder 10 and the magnet 20 as a target to be guided, and guides the target so as to move along the guide portion 31. When the guide portion 31 is a rail, the target to be guided is engaged with the guide portion 31 so as to move on the rail. When the guide portion 31 is a guide groove, at least a part of the target to be guided is disposed in the guide groove and is engaged with the guide portion 31 so as to move in the guide groove. The guide portion 31 may guide the holder 10 instead of the magnet 20. The guide portion 31 may guide both the magnet 20 and the holder 10.

In the configuration example shown in FIG. 10, the agitator 40 is connected to the holder 10 so as to move with the holder 10, and is configured to agitate the sample in the container 90 when the holder 10 is driven. Thus, the agitator 40 can be moved integrally with the holder 10 by the movement mechanism 30. Therefore, the device configuration can be simplified as compared to, for example, a configuration in which the holder 10 is connected to a fixed agitator 40 when moving to the separate position 62 (see FIG. 1).

In the configuration example shown in FIG. 10, the magnet 20 is disposed laterally to the holder 10 so as to approach or separate from the side surface of the container 90 held by the holder 10. Thus, the magnetic particles 82 can be magnetically collected on the side surface of the container 90 in the approach position 61. Therefore, when the liquid component 88 is sucked by the suction tube 50, the liquid component 88 on the bottom surface of the container 90 can be satisfactorily sucked without sucking the collected magnetic particles 82, in contrast to the case where the magnetic particles 82 are collected on the bottom surface side of the container 90 as shown in FIG. 6B. Thus, the magnetic particles 82 can be effectively separated from the liquid component 88.

### [Specific configuration example of sample analyzer]

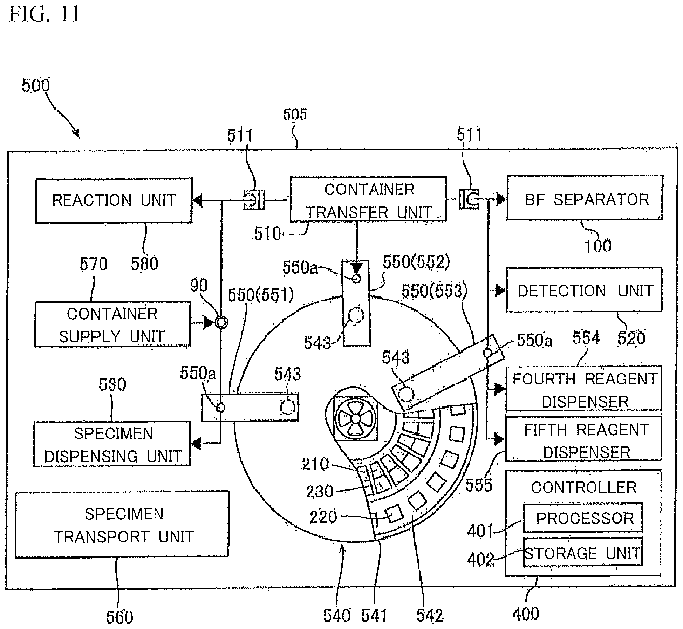

Next, a specific configuration example of the sample analyzer 500 will be described in detail with reference to FIG. 11 to FIG. 17. In the example shown in FIG. 11 to FIG. 17, the sample analyzer 500 is an immunoassay apparatus configured to detect a test substance in a specimen by using an antigen-antibody reaction. The specimen is serum or plasma.

The sample analyzer 500 includes the container transfer unit 510, the BF separator 100, and the detection unit 520. In the configuration example shown in FIG. 11, the sample analyzer 500 includes a housing 505, a specimen dispensing unit 530, a reagent cooling box 540, a reagent dispenser 550, a specimen transport unit 560, a container supply unit 570, and a reaction unit 580. The sample analyzer 500 further includes a controller 400 for controlling the respective components described above.

The housing 505 has a box shape capable of housing the respective components of the sample analyzer 500. The housing 505 may be configured to house the respective components of the sample analyzer 500 on a single stage. Alternatively, the housing 505 may have a multi-stage structure in which a plurality of stages are provided in the up-down direction, and the respective components of the sample analyzer 500 may be disposed on the stages allocated thereto.

The specimen transport unit 560 is configured to transport a specimen collected from a subject to a suction position of the specimen dispensing unit 530. The specimen transport unit 560 can transport, to a predetermined specimen suction position, a rack on which a plurality of test tubes each storing a specimen therein are placed.

The specimen dispensing unit 530 can suck the specimen transported by the specimen transport unit 560, and dispense the sucked specimen into the container 90. The specimen dispensing unit 530 includes: a pipette that is connected to a fluid circuit and performs suction and discharge; and a movement mechanism for moving the pipette. The specimen dispensing unit 530 attaches a dispensing tip, which is set in a tip feeder (not shown), to the tip of the pipette, and sucks a predetermined amount of specimen in the transported test tube, into the dispensing tip. The specimen dispensing unit 530 dispenses the sucked specimen into the container 90 disposed in a predetermined specimen dispensing position. After the dispensing, the specimen dispensing unit 530 removes the dispensing tip from the tip of the pipette, and discards the dispensing tip.

The container supply unit 570 can store therein a plurality of containers 90 that have not been used. The container supply unit 570 can supply unused empty containers 90 one by one to the container transfer unit 510, in a predetermined container supply position.

The container transfer unit 510 can transfer the containers 90. The container transfer unit 510 obtains an empty container 90 from the container supply position, and sequentially transports the container 90 to the processing positions of the BF separator 100, the specimen dispensing unit 530, the reagent dispenser 550, the reaction unit 580, the detection unit 520, and the like. For example, the container transfer unit 510 includes: catchers 511 which grip the container 90 or a holder having a hole in which the container 90 is set; and a movement mechanism for moving the catcher 511 or the holder. The movement mechanism is moved in a single axial direction or a plurality of axial directions by a direct acting mechanism linearly movable in one or a plurality of directions. The movement mechanism is movable in three orthogonal directions, i.e., the up-down direction and the two horizontal directions, for example. The movement mechanism may include an arm mechanism that horizontally rotates around a rotary axis, or an articulated robot mechanism. One or a plurality of container transfer units 510 are provided according to the arrangement of the processing positions of the specimen dispensing unit 530, the reagent dispenser 550, the reaction unit 580, the detection unit 520, and the like in the housing 505.

The reaction unit 580 includes a heater and a temperature sensor, and heats the sample stored in the container 90 while holding the container 90, to cause the sample to react. The heating causes the specimen and a reagent stored in the container 90 to react. One or a plurality of reaction units 580 are provided in the housing 505. The reaction unit 580 may be fixedly disposed in the housing 505, or may be movable in the housing 505. When the reaction unit 580 is movable, the reaction unit 580 can also function as a part of the container transfer unit 510.

In the configuration example shown in FIG. 11, the reagent cooling box 540 has a container holder 542 and a cooling mechanism in a case 541. The container holder 542 holds reagent containers. The cooling mechanism keeps reagents in the reagent containers cool at a predetermined temperature.

The case 541 has an inner space defined by a circular case upper surface, a circular case bottom surface, and a cylindrical case side surface. The case 541 includes a heat insulating material that thermally insulates the inside of the case 541 from the outside. Thus, the reagent containers can be kept at a cool temperature. The reagent cooling box 540 has a plurality of holes 543 which allow the reagent dispenser 550 to enter the inside of the reagent cooling box 540.

The container holder 542 is formed so as to hold a plurality of reagent containers arranged in a circumferential direction. The container holder 542 can hold a plurality of reagent containers arranged in a radial direction. That is, on the container holder 542, a plurality of lines of reagent containers arranged in the circumferential direction are concentrically arranged in the radial direction. The container holder 542 can rotate the plurality of concentric lines of reagent containers independently from each other in the circumferential direction. The positions of the plurality of holes 543 in the radial direction are different from each other such that each hole 543 corresponds to any of the concentric lines of reagent containers. Thus, the container holder 542 can dispose, at a position directly below each of the plurality of holes 543 provided in different reagent dispensers 550, a desired reagent container selected from the corresponding line of reagent containers. As a result, the reagent in the reagent container disposed directly beneath the hole 543 is sucked by the reagent dispenser 550. In the container holder 542, reagent containers 210, 220, and 230 respectively storing therein an R1 reagent, an R2 reagent, and an R3 reagent described later are set.

The reagent dispenser 550 sucks the reagent in the reagent container, and dispenses the sucked reagent into the container 90. The reagent dispenser 550 can move a pipette 550a for sucking and discharging the reagent, in the horizontal direction between the hole 543 and a predetermined reagent dispensing position. In addition, the reagent dispenser 550 can move the pipette 550a in the up-down direction, so that the pipette 550a can penetrate through the hole 543 from above to enter the inside of the reagent container, and the pipette 550a can be withdrawn to a position above the hole 543. The pipette 550a is connected to a fluid circuit (not shown), sucks a predetermined amount of reagent from the reagent container held by the container holder 542, and dispenses the reagent into the container 90 transported to the reagent dispensing positon.

For example, three reagent dispensers 550 are provided for dispensing the R1, R2, and R3 reagents, respectively. One reagent dispenser 550 may dispense a plurality of kinds of reagents. In the configuration example shown in FIG. 11, the reagent dispensers 550 include: a first reagent dispenser 551 for dispensing the R1 reagent into the reagent container 210; a second reagent dispenser 552 for dispensing the R2 reagent into the reagent container 220; and a third reagent dispenser 553 for dispensing the R3 reagent into the reagent container 230. The reagent dispensers 550 further include: a fourth reagent dispenser 554 for dispensing an R4 reagent; and a fifth reagent dispenser 555 for dispensing an R5 reagent.

The first reagent dispenser 551 can move the pipette 550a between a predetermined R1 reagent dispensing position and the hole 543 for sucking the R1 reagent from the reagent container 210 in which the R1 reagent is stored. The second reagent dispenser 552 can move the pipette 550a between a predetermined R2 reagent dispensing position and the hole 543 for sucking the R2 reagent from the reagent container 220 in which the R2 reagent is stored. The third reagent dispenser 553 can move the pipette 550a between a predetermined R3 reagent dispensing position and the hole 543 for sucking the R3 reagent from the reagent container 230 in which the R3 reagent is stored. The fourth reagent dispenser 554 and the fifth reagent dispenser 555 are disposed at positions away from the reagent cooling box 540. The fourth reagent dispenser 554 and the fifth reagent dispenser 555 are respectively connected to reagent containers (not shown) in which the R4 reagent and the R5 reagent are stored, via liquid feed tubes, and therefore can discharge the reagents into the container 90 transported by the container transfer unit 510.

The BF separator 100 has a function of executing a BF separation process for separating a liquid phase from a solid phase in the container 90. In the sample analyzer 500, one or a plurality of BF separators 100 are provided. In a state where magnetic particles 82 having an immune complex formed thereon as described later are magnetically collected, the BF separator 100 causes the suction tube 50 to suck a liquid component in the container 90, and causes the discharge tube 51 to supply a washing liquid into the container 90. Each of the suction tube 50 and the discharge tube 51 is connected to a fluid circuit (not shown). Thus, an unnecessary substance contained in the liquid component can be separated and removed from the magnetic particles 82.

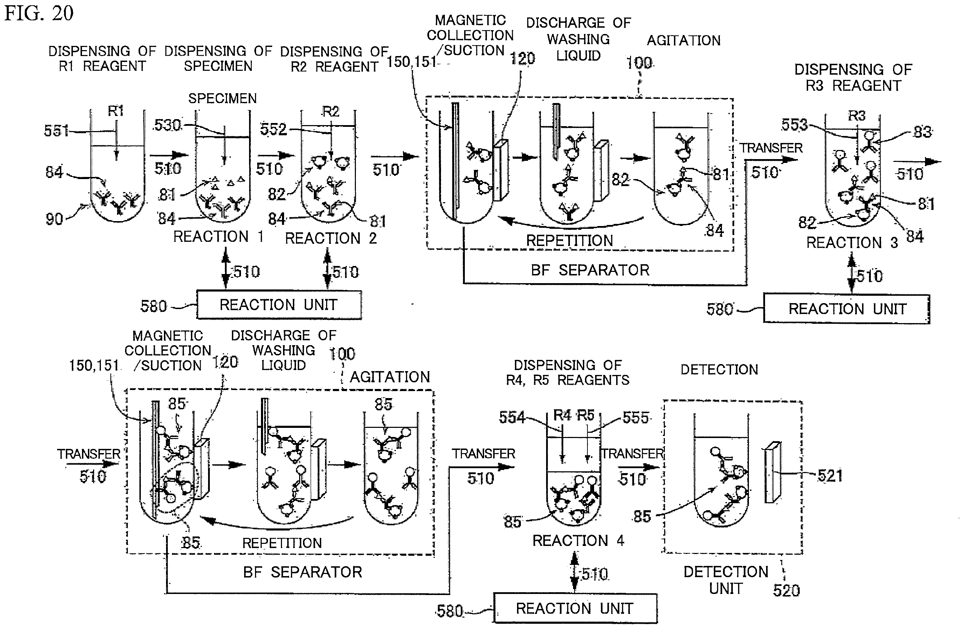

The detection unit 520 includes a photodetector 521 (see FIG. 20) such as a photomultiplier. The detection unit 520 obtains, by using the photodetector 521, light generated in the reaction process between a luminescent substrate and a labeled antibody binding to an antigen of a specimen having been subjected to various processes, thereby measuring the quantity of the antigen contained in the specimen.

The controller 400 includes a processor 401 such as a CPU, and a storage unit 402 such as a ROM, a RAM, or a hard disk. The processor 401 executes a control program stored in the storage unit 402 to function as a controller for the sample analyzer 500. The controller 400 controls the operations of the respective components of the sample analyzer 500 described above.

### (Detailed configuration ofBF separator)

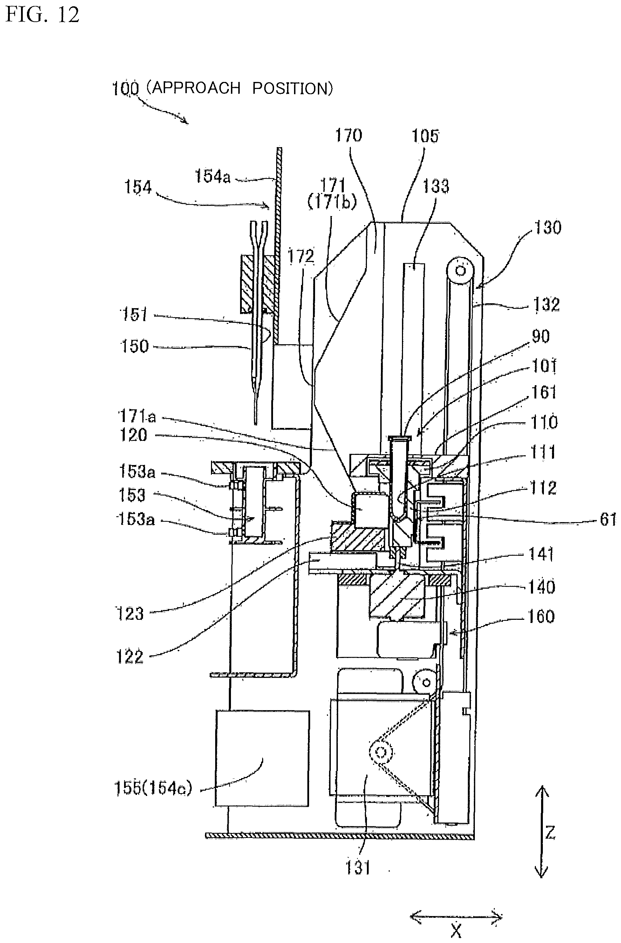

With reference to FIG. 12 to FIG. 17, a configuration example of the BF separator 100 in the sample analyzer 500 will be described in detail.

In the configuration example shown in FIG. 12 to FIG. 14, the BF separator 100 includes a holder 110, a magnet 120, a movement mechanism 130, an agitator 140, a suction tube 150, and a discharge tube 151.

### <Movement mechanism>

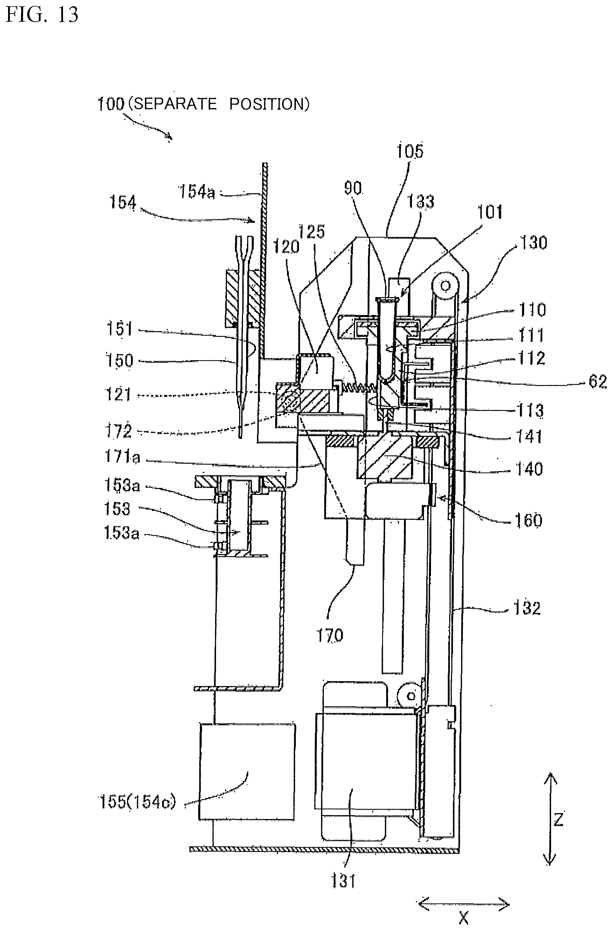

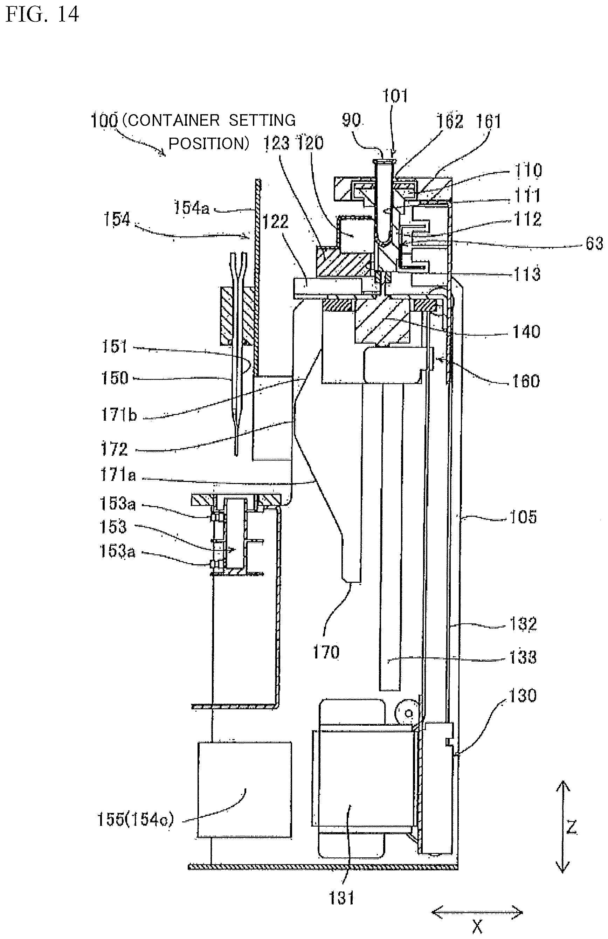

In the configuration example shown in FIG. 12 to FIG. 14, the movement mechanism 130 is configured to move the holder 110 and the magnet 120 in the up-down direction. The movement mechanism 130 is configured to move both the holder 110 and the magnet 120 in conjunction with one another, to an approach position 61 (see FIG. 12), a separate position 62 (see FIG. 13), and a container setting position 63 (see FIG. 14) where setting and removal of a container 90 are performed. The approach position 61, the separate position 62, and the container setting position 63 have different heights. In the configuration example shown in FIG. 12 to FIG. 14, the container setting position 63, the separate position 62, and the approach position 61 are arranged in this order from the top.

That is, in the configuration example shown in FIG. 12 to FIG. 14, the container setting position 63 is disposed above the approach position 61 and the separate position 62. Thus, the suction tube 150 performing suction of the liquid component in the approach position 61 and the agitator 140 performing agitation of the sample in the separate position 62 are less likely to become obstacles when setting/removal of the container 90 is performed in the container setting position 63. Therefore, it is possible to easily avoid interference with the components related to setting/removal of the container 90 in/from the holder 110.

The movement mechanism 130 includes a common drive unit 131 for moving both the holder 110 and the magnet 120. In this case, since a drive source can be shared by the holder 110 and the magnet 120, the configuration of the movement mechanism 130 can be simplified. As a result, the installation area of the BF separator 100 can be further reduced.

In the configuration example shown in FIG. 12 to FIG. 14, the BF separator 100 includes a movement base 160 on which the holder 110 and the magnet 120 are provided. The movement mechanism 130 causes the drive unit 131 to move the movement base 160. Thus, by only moving the movement base 160, the movement mechanism 130 can move both the holder 110 and the magnet 120 in conjunction with one another. Therefore, the configuration of the movement mechanism 130 can be further simplified.

In the configuration example shown in FIG. 12 to FIG. 14, the movement mechanism 130 is configured to move the holder 110, the magnet 120, and the agitator 140 in conjunction with one another. Thus, not only the holder 110 and the magnet 120 but also the agitator 140 can be moved in conjunction with one another by the movement mechanism 130. Therefore, the device configuration for causing the agitator 140 to agitate the sample in the container 90 held by the holder 110 in the separate position 62 can be simplified.

Specifically, the agitator 140 is provided on the movement base 160, and configured to agitate the sample in the container 90 with the holder 110 being driven. Thus, since not only the holder 110 and the magnet 120 but also the agitator 140 can be provided on the movement base 160, downsizing of the BF separator 100 can be effectively achieved. In contrast to the case where, for example, an agitation rod or the like is inserted in the container 90, the agitator 140 only has to vibrate the holder 110. Thus, the configuration of the agitator 140 is simplified, and downsizing of the agitator 140 is achieved.

Specifically, in the configuration example shown in FIG. 12 to FIG. 14, the movement mechanism 130 includes the drive unit 131, a transmission mechanism 132, and a guide rail 133. The drive unit 131 is disposed in a lower part of the BF separator 100. The drive unit 131 is a motor such as a stepping motor or a servo motor.

The transmission mechanism 132 connects an output shaft of the drive unit 131 to the movement base 160 to transmit a driving force of the drive unit 131 to the movement base 160. The drive unit 131 moves the movement base 160, via the transmission mechanism 132, to each of the approach position 61, the separate position 62, and the container setting position 63. In the configuration example shown in FIG. 12 to FIG. 14, the transmission mechanism 132 is a belt pulley mechanism. The movement base 160 is fixed to a predetermined position on the belt of the transmission mechanism 132 via a bracket, and is moved with movement of the belt.

The transmission mechanism 132 may have a well-known configuration. For example, a rack-and-pinion mechanism or a direct acting mechanism using a ball shaft and a ball nut can be adopted. The movement mechanism 130 may have a configuration, such as a linear motor, which directly applies a driving force generated by the drive unit 131 to the movement base 160. In this case, the transmission mechanism 132 is not necessary.

The guide rail 133 is provided so as to linearly extend in the up-down direction. The movement base 160 is provided with a slider (not shown) which linearly moves along the guide rail 133. The movement mechanism 130 drives the movement base 160 by using the drive unit 131 and the transmission mechanism 132 to linearly move the movement base 160 in the up-down direction along the guide rail 133. The drive unit 131, the guide rail 133, and the transmission mechanism 132 are mounted to a chassis 105 of the BF separator 100.

In the configuration example shown in FIG. 12 to FIG. 14, the BF separator 100 includes a guide portion 170 which comes into contact with at least one of the holder 110 and the magnet 120 being moved by the movement mechanism 130, and causes the holder 110 and the magnet 120 to approach each other or separate from each other.

The BF separator 100 further includes an urging member 125 (see FIG. 13) which urges the holder 110 and the magnet 120 so as to approach each other. The guide portion 170 is configured to cause the holder 110 and the magnet 120 to separate from each other against an urging force of the urging member 125, and cause the holder 110 and the magnet 120 to approach each other by the urging force of the urging member 125. Therefore, the guide portion 170 only has to be formed to separate the holder 110 and the magnet 120 from each other against the urging force of the urging member 125, and it is not necessary to provide both a guide for causing the holder 110 and the magnet 120 to separate from each other and a guide for causing the holder 110 and the magnet 120 to approach each other. Thus, the configuration of the guide portion 170 can be simplified.

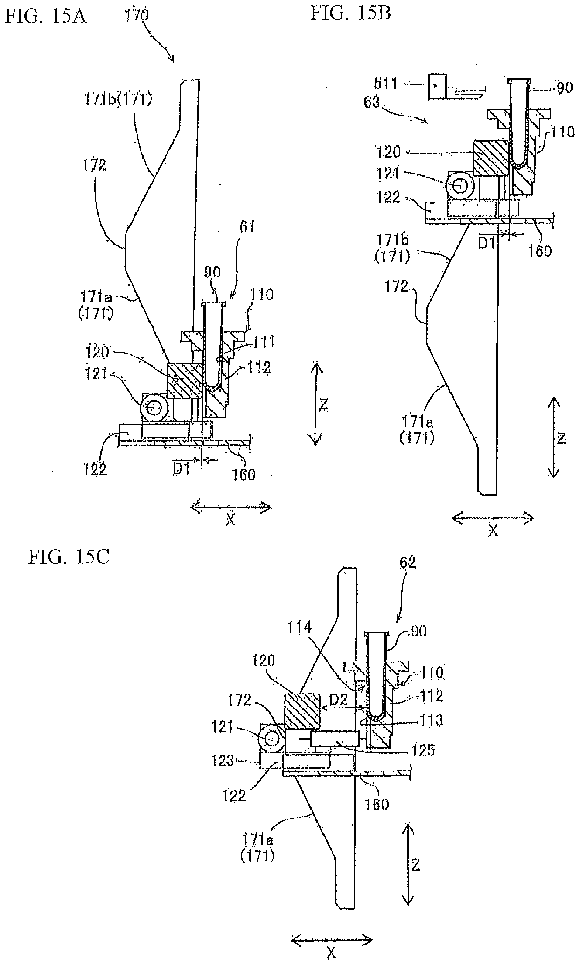

FIG. 15A to FIG. 15C schematically show the guide portion 170, the holder 110, and the magnet 120 in an enlarged manner. The guide portion 170 has a surface 171 which inclines with respect to the movement direction from the approach position 61 and the separate position 62 so as to cause the holder 110 and the magnet 120 to separate from each other as they approach the separate position 62 from the approach position 61. At least one of the holder 110 and the magnet 120 includes a rotating body 121 which rolls on the inclined surface 171. The rotating body 121 rolling on the inclined surface 171 of the guide portion 170 allows at least one of the holder 110 and the magnet 120 to relatively move in a direction different from the movement direction from the approach position 61 to the separate position 62 caused by the movement mechanism 130. As a result, the holder 110 and the magnet 120 can be caused to approach each other or separate from each other by the simple structure using the inclined surface 171 and the rotating body 121.

In the configuration example shown in FIG. 15A to FIG. 15C, the magnet 120 is provided with the rotating body 121. The rotating body 121 is a roller having a circular outer peripheral surface. The rotating body 121 is disposed at a position where the rotating body 121 can come into contact with the guide portion 170 with the up-down movement of the movement base 160 caused by the movement mechanism 130. The rotating body 121 is movable in the horizontal direction together with the magnet 120.

In the configuration example shown in FIG. 15A to FIG. 15C, the guide portion 170 is a plate member having the inclined surface 171. The surface 171 is inclined with respect to the up-down direction so as to be away from the holder 110 toward the separate position 62. In FIG. 15A to FIG. 15C, since the holder 110 is disposed on the right side in each figure, the surface 171 is inclined so as to protrude leftward in each figure. In addition, in the configuration example shown in FIG. 15A to FIG. 15C, the guide portion 170 includes an inclined surface 171a between the approach position 61 and the separate position 62 and an inclined surface 171b between the container setting position 63 and the separate position 62. The guide portion 170 has a flat surface 172 linearly extending in the up-down direction, at a height position corresponding to the separate position 62. The flat surface 172 is connected to the inclined surface 171a at a lower end thereof, and is connected to the inclined surface 171b at an upper end thereof.

As shown in FIG. 15A and FIG. 15B, in the approach position 61 and the container setting position 63, the rotating body 121 is disposed at a position away from the guide portion 170 in the horizontal direction. The rotating body 121 is not in contact with the guide portion 170 in the approach position 61 and the container setting position 63. The rotating body 121 is disposed at a position overlapping the inclined surface 171 in the up-down direction. In the approach position 61 and the container setting position 63, the magnet 120 and the holder 110 are close to each other over a distance D1.

When the movement base 160 is raised from the approach position 61 toward the separate position 62, the rotating body 121 comes into contact with the inclined surface 171a. Since the magnet 120 is urged toward the holder 110 by the urging member 125, the rotating body 121 moves as if climbing the inclined surface 171a while being kept in contact with the inclined surface 171a. As a result, the magnet 120 horizontally moves so as to be away from the holder 110, against the urging force of the urging member 125.

As shown in FIG. 15C, when the movement base 160 has reached the separate position 62, the rotating body 121 comes into contact with the flat surface 172, and the urging force by the urging member 125 is supported by the flat surface 172. As a result, in the separate position 62, the magnet 120 and the holder 110 are away from each other over a distance D2 (>D1) greater than the distance D1.

Thus, the movement mechanism 130 is configured to move the holder 110 and the magnet 120 in the up-down direction, and the holder 110 and the magnet 120 relatively move so as to approach each other or separate from each other in the direction intersecting the up-down direction, in conjunction with the movement in the up-down direction. In the approach position 61, the holder 110 and the magnet 120 are close to each other over the distance D1 that allows the magnetic particles 82 in the container 90 to be magnetically collected. In the separate position 62, the holder 110 and the magnet 120 are away from each other over the distance D2 that allows magnetic collection of the magnetic particles 82 in the container 90 to be canceled.

### <Holder, magnet, agitator>

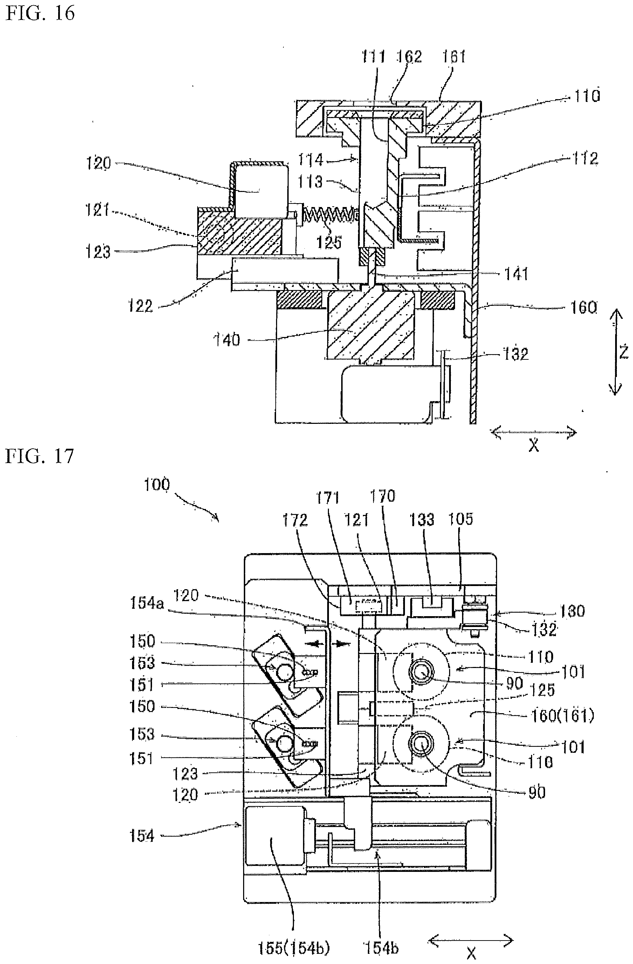

As shown in FIG. 16, the magnet 120 is disposed laterally to the holder 110 so as to approach or separate with respect to the side surface of the container 90 held by the holder 110.

Specifically, the magnet 120 is provided to be movable in the horizontal direction, on the guide rail 122 provided on the movement base 160. Thus, on the movement base 160, the magnet 120 can move relative to the holder 110 in a direction approaching or separating from the holder 110. The magnet 120 is connected to one end of the urging member 125 provided in the horizontal direction. The other end of the urging member 125 is connected to a fixing portion disposed at a position on the holder 110 side on the movement base 160. In the configuration example shown in FIG. 16, the urging member 125 is a tension coil spring, and applies an urging force to the magnet 120 in a direction approaching the holder 110. The urging force causes the magnet 120 to approach or separate from the side surface of the container 90 held by the holder 110.

In the configuration example shown in FIG. 16, the holder 110 is configured to expose the side surface, on the magnet 120 side, of the held container 90, such that the magnet 120 and the container 90 held by the holder 110 are close to each other in the approach position 61. Thus, in the approach position 61, the magnet 120 and the container 90 held by the holder 110 can be sufficiently close to each other, whereby the magnetic particles 82 can be effectively magnetically collected.

Specifically, the holder 110 includes: the holding hole 111 for holding the container 90; and an open side surface 113 having, formed therein, an opening that exposes the side surface of the container 90 disposed in the holding hole 111. That is, the holder 110 has a peripheral wall portion 112 that partitions the holding hole 111, and a side surface, on the magnet 120 side, of the peripheral wall portion 112 is the open side surface 113. As shown in FIG. 12, in the approach position 61, the magnet 120 is disposed in contact with the open side surface 113. The urging force to the holder 110 side caused by the urging member 125 is supported by the open side surface 113 being in contact with the magnet 120. That is, in the approach position 61, the magnet 120 is held while being pressed against the open side surface 113 by the urging force.

Thus, in the approach position 61, the magnet 120 can be brought as close as possible to the container 90 in the holding hole 111. In addition, since positioning of the magnet 120 is achieved when the magnet 120 comes into contact with the open side surface 113, the distance between the magnet 120 and the container 90 in the approach position 61 can be kept constant, and the state of magnetic collection during suction of the liquid component by the suction tube 150 can be stabilized without variation.

As shown in FIG. 14, also in the container setting position 63, like in the approach position 61, the magnet 120 is located in contact with the open side surface 113. Since the magnet 120 is supported in contact with the open side surface 113, the rotating body 121 shown in FIG. 15A to FIG. 15C is not in contact with the guide portion 170 at a position away from the guide portion 170 in the horizontal direction, in each of the approach position 61 and the container setting position 63.

The peripheral wall portion 112 of the holder 110 has a substantially cylindrical shape. An upper end of the peripheral wall portion 112 is opened to form the holding hole 111, and a lower end of the peripheral wall portion 112 is closed. An upper end portion of the peripheral wall portion 112 is formed in a flange shape extending outward in the radial direction. The open side surface 113 of the peripheral wall portion 112 is a flat surface. An opening 114 communicating with the holding hole 111 is formed at the open side surface 113, and the container 90 disposed in the holding hole 111 is exposed through the opening 114.

In the configuration example shown in FIG. 16, the agitator 140 is connected to the holder 110 so as to move with the holder 110. The agitator 140 is configured to agitate the sample in the container 90 by driving the holder 110.

Specifically, the agitator 140 is a rotary motor mounted to the movement base 160. The agitator 140 is located directly below the holder 110, and an output shaft 141 extending upward is mounted on the bottom of the holder 110. The output shaft 141 is connected to the holder 110 at a position slightly eccentric from the center of the holder 110 in a horizontal cross section. Thus, the agitator 140 eccentrically rotates the holder 110 around the rotary shaft in the up-down direction. As a result, the agitator 140 vibrates and agitates the sample in the container 90 held by the holder 110. An upper end portion of the holder 110 is covered with an upper face cover 161 fixed to the movement base 160, so that a part of the holder 110 rotated by the agitator 140 is not exposed. The upper face cover 161 has a through-hole 162 formed at a position overlapping the holding hole 111, and the container 90 is set in the holding hole 111 through the through-hole 162.

### <Processing port>

FIG. 17 is a plan view of the BF separator 100 shown in FIG. 12 to FIG. 14. In the configuration example shown in FIG. 17, the BF separator 100 includes a plurality of processing ports 101 each holding a container 90 and performing a BF separation process. That is, a plurality of processing ports 101 each including a holder 110 and a magnet 120 are provided. In the configuration example shown in FIG. 17, the BF separator 100 includes two processing ports 101. The BF separator 100 includes suction tubes 150 and discharge tubes 151 as many as the processing ports 101. The number of processing ports 101 may be one, or three or more. The number of suction tubes 150 and the number of discharge tubes 151 each may be one, or three or more. The number of suction tubes 150 and the number of discharge tubes 151 need not be the same, and may be different from each other. The plurality of processing ports 101 may share a suction tube 150 and a discharge tube 151. That is, suction tubes 150 and discharge tubes 151, each less in number than the processing ports 101, may be provided.

In the configuration example shown in FIG. 17, a plurality of processing ports 101 each including a holder 110 and a magnet 120 are provided on the movement base 160. Thus, a plurality of containers 90 can be set in the single movement base 160, and samples in the plurality of containers 90 can be subjected to the BF separation process. Therefore, the installation area of the BF separator 100 can be effectively reduced. Also, in the configuration having the plurality of processing ports 101, the holder 110 and the magnet 120 included in each processing port 101 can be moved together to the approach position 61 and to the separate position 62 by only providing a single common movement mechanism 130. Therefore, in particular, when a plurality of processing ports 101 are provided, the processing ports 101 can share the movement mechanism 130, whereby the installation area of the apparatus can be effectively reduced.

In the configuration example shown in FIG. 17, each of the two processing ports 101 includes an agitator 140 (see FIG. 16) connected to the holder 110. Each holder 110 is independently subjected to agitation by the corresponding agitator 140.

In the configuration example shown in FIG. 17, two magnets 120 are provided on a common setting portion 123, and can integrally move in the horizontal direction on a common guide rail 122 (see FIG. 16). One urging member 125 is connected to the setting portion 123, and urges the two magnets 120 at the same time.

### <Suction tube, discharge tube>

In the configuration example shown in FIG. 12 to FIG. 14, the suction tube 150 is provided so as to extend in the up-down direction. The suction tube 150 has a suction hole at a lower end thereof, and is connected, at an upper end thereof, to a fluid circuit (not shown) through a flow path (not shown) such as a liquid feed tube. Thus, the suction tube 150 can suck the liquid component in the container 90 from the lower end.

The discharge tube 151 is provided so as to extend in the up-down direction. The discharge tube 151 has a discharge hole at a lower end thereof, and is connected, at an upper end thereof, to a washing liquid container (not shown) through a fluid circuit (not shown) and a flow path (not shown) such as a liquid feed tube. Thus, the discharge tube 151 can discharge the washing liquid from the lower end into the container 90.

In the configuration example shown in FIG. 12 to FIG. 14, the BF separator 100 is provided with a washer 153 for washing the suction tube 150. In the configuration having the discharge tube 151, the washer 153 may wash the discharge tube 151. The washer 153 is disposed at substantially the same height position as the holder 110 in the approach position 61. The suction tube 150 is configured to move between the container 90 held by the holder 110 in the approach position 61, and the washer 153. Thus, since the holder 110 in the approach position 61 and the washer 153 are disposed at substantially the same height, the movement route of the suction tube 150 between the washer 153 and the container 90 in the approach position 61 can be simplified. Therefore, downsizing of the BF separator 100 can be achieved. As described above, in the configuration example shown in FIG. 12, the washer 153 and the holder 110 in the approach position 61 are disposed side by side in the horizontal direction. The washer 153 is disposed adjacent to the movement base 160 in the approach position 61.

The washer 153 has a cylindrical shape with an opened upper end and a closed lower end, so that a washing liquid can be stored therein. Two ports 153a for supply and discharge of the washing liquid are formed in a side surface portion of the washer 153, and in communication with the inside of the washer 153. The two ports 153a are respectively connected to the washing liquid container and a waste liquid container via fluid circuits (not shown). In washing, the suction tube 150 and the discharge tube 151 enter the inside of the washer 153 from above through the opening of the washer 153. Then, the washing liquid is supplied into the washer 153, and the suction tube 150 is washed. Since the discharge tube 151 is not likely to come into contact with the sample in the container 90, washing the discharge tube 151 is not always necessary. However, the discharge tube 151 may be periodically washed.

In the configuration example shown in FIG. 12 to FIG. 14, the suction tube 150 and the discharge tube 151 are attached together to a nozzle movement mechanism 154 so that these tubes 150 and 151 can enter the inside of the container 90 together. As shown in FIG. 17, two sets of the suction tube 150 and the discharge tube 151 are mounted to the nozzle movement mechanism 154 so as to correspond to the two processing ports 101. In addition, as many washers 153 as the processing ports 101 are provided. With the nozzle movement mechanism 154, the suction tube 150 and the discharge tube 151 of each processing port 101 can enter the washer 153 and the container 90 held by the corresponding holder 110, and can withdraw from the washer 153 and the container 90.

Specifically, as shown in FIG. 12 and FIG. 17, the nozzle movement mechanism 154 includes: a tube support portion 154a which is movable while supporting the two sets of the suction tube 150 and the discharge tube 151; a horizontal movement mechanism 154b (see FIG. 17) which moves the tube support portion 154a in the horizontal direction; and an up-down movement mechanism 154c (see FIG. 12) which moves the tube support portion 154a in the up-down direction.

The tube support portion 154a is formed in a plate shape extending in the up-down direction. The horizontal movement mechanism 154b causes the suction tube 150 and the discharge tube 151 supported by the tube support portion 154a to linearly move in the horizontal direction between the container 90 held by the corresponding holder 110, and the washer 153. The up-down movement mechanism 154c causes the suction tube 150 and the discharge tube 151 supported by the tube support portion 154a to linearly move in the up-down direction. The up-down movement mechanism 154c causes the suction tube 150 and the discharge tube 151 to move in the up-down direction to the inside of the container 90 held in the approach position 61 and to a withdrawal position above the container 90 held by the approach position 61. The up-down movement mechanism 154c causes the suction tube 150 and the discharge tube 151 to move in the up-down direction to a washing position inside the washer 153 and to a withdrawal position above the washer 153. Each of the horizontal movement mechanism 154b and the up-down movement mechanism 154c is, for example, a direct acting mechanism composed of a drive source 155 such as a stepping motor or a servo motor, and a transmission mechanism.

### <Approach position, separate position, container setting position>

As shown in FIG. 12 to FIG. 14 and FIG. 15A to FIG. 15C, the approach position 61 and the separate position 62 are positions that allow at least one of the holder 110 and the magnet 120 to be disposed on a straight line in the up-down direction. Thus, at least one of the holder 110 and the magnet 120 need not be moved in the horizontal direction, whereby a space, in the horizontal direction, necessary for movement between the approach position 61 and the separate position 62 can be reduced. Therefore, the installation area of the BF separator 100 can be effectively reduced.

In the configuration example shown in FIG. 12 to FIG. 14 and FIG. 15A to FIG. 15C, the holder 110 is disposed on a straight line in the up-down direction, in the approach position 61, the separate position 62, and the container setting position 63. Therefore, in the BF separator 100, the container 90 held by the holder 110 is not moved in the horizontal direction except when it is vibrated during agitation. Therefore, for example, in contrast to the case where, instead of the magnet 120, the holder 110 is horizontally moved with the movement in the up-down direction, the sample in the container 90 is prevented from spilling out even if the moving speed of the movement base 160 in the up-down direction by the movement mechanism 130 is increased.

As shown in FIG. 12 and FIG. 15A to FIG. 15C, the approach position 61 is located below the separate position 62. Here, the suction tube 150 is required to have a certain length in the up-down direction in order to suck the liquid component from the container 90 in the approach position 61, and a relatively large space tends to be required in the up-down direction because the suction tube 150 is connected to a liquid feed tube or the like to transfer the sucked liquid component. Therefore, when the approach position 61 is located on the lower side while the separate position 62 is located on the upper side, a space, in the up-down direction, required for the suction tube 150 to suck the liquid component can be ensured by using the space for moving the holder 110 in the approach position 61 to the upper separate position 62. Thus, downsizing of the BF separator 100 can be achieved by the effective use of the space between the approach position 61 and the separate position 62.

As shown in FIG. 15A to FIG. 15C, in the approach position 61, the holder 110 and the magnet 120 approach each other over the distance D1 that allows the magnetic particles 82 in the container 90 to be magnetically collected. In the separate position 62, the holder 110 and the magnet 120 are away from each other over the distance D2 that allows magnetic collection of the magnetic particles 82 in the container 90 to be canceled. Thus, when suction of the liquid component by the suction tube 150 is performed in the approach position 61, suction of the magnetic particles 82 in the container 90 together with the liquid component can be effectively inhibited by the magnetic collection. In addition, since the magnetic collection of the magnetic particles 82 is canceled when the sample is agitated by the agitator 140 in the separate position 62, an unnecessary substance adhered to the magnetic particles 82 can be effectively dispersed in the liquid component.

In the container setting position 63, the holder 110 and the magnet 120 approach each other over the distance D1 that allows the magnetic particles 82 in the container 90 to be magnetically collected. Therefore, magnetic collection of the magnetic particles 82 can be started from when the container 90 is set in the holder 110 in the container setting position 63. Thus, the time required for magnetic collection of the magnetic particles 82 can be reduced as compared to the case where magnetic collection is performed only in the approach position 61.

In the configuration example shown in FIG. 12 to FIG. 17, the movement mechanism 130 moves the holder 110 and the magnet 120 to the approach position 61 and to the separate position 62, a plurality of number of times, so as to repeat: suction of the liquid component by the suction tube 150 and discharge of the washing liquid by the discharge tube 151 in the approach position 61; and agitation of the sample by the agitator 140 in the separate position 62. Thus, suction of the liquid component and discharge of the washing liquid, and agitation of the sample can be repeated the plurality of number of times, thereby enhancing separation performance in the BF separation process. Even in the case where suction of the liquid component and discharge of the washing liquid, and agitation of the sample are repeated the plurality of number of times, it is only necessary to cause the magnet 120 and the holder 110 holding the container 90 to reciprocate between the approach position 61 and the separate position 62. Since an operation to take out the container 90 from the holder 110 is not required, the device configuration can be simplified, and the BF separation process can be performed in a short time. The number of times the suction of the liquid component and discharge of the washing liquid, and agitation of the sample may be 2, 3, or 4, for example. The number of repetitions may be 5 or more.

### <Operation for BF separation process>

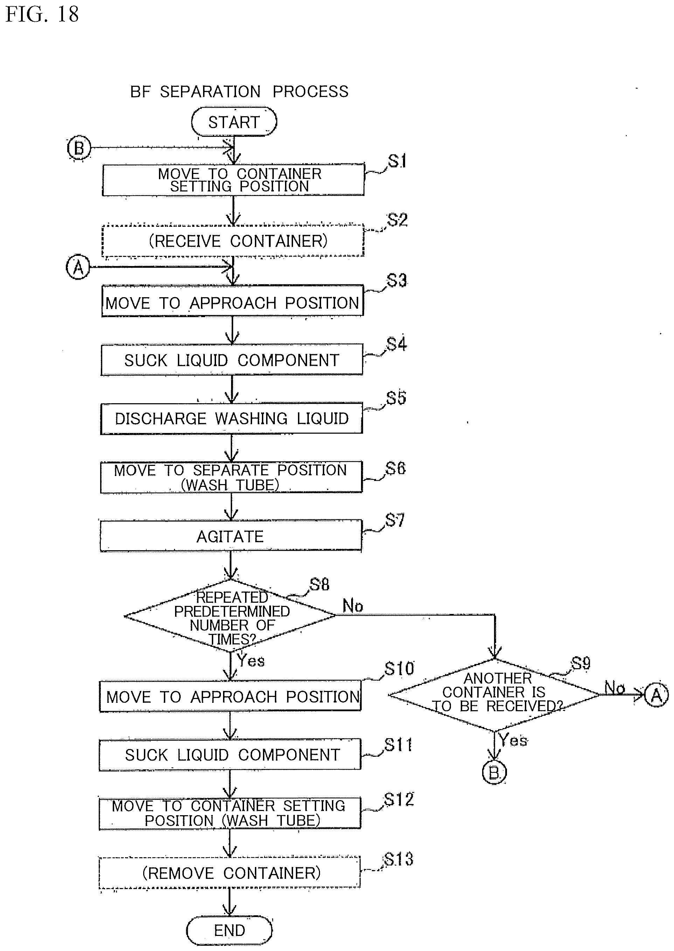

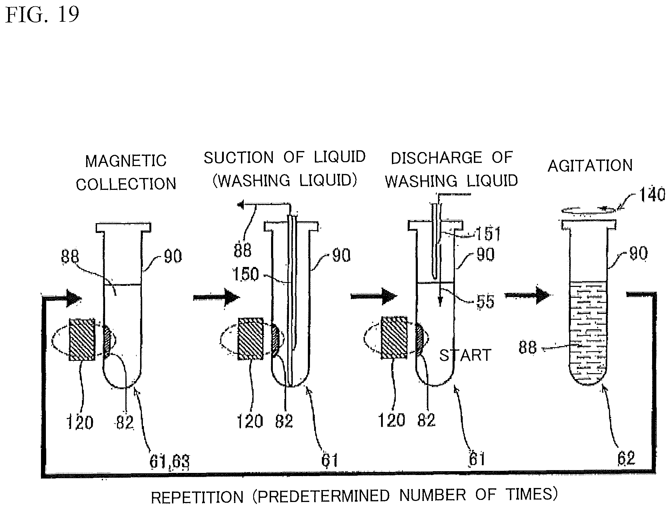

With reference to FIG. 18 and FIG. 19, the operation of the BF separator 100 for the BF separation process will be described. The process in each step shown in FIG. 18 is controlled by the controller 400.

In step S1, the controller 400 causes the movement mechanism 130 to move the movement base 160 to the container setting position 63. In step S2, the controller 400 causes the container transfer unit 510 (see FIG. 11) to set a container 90 in a holder 110 of either processing port 101. In the container setting position 63, the holder 110 receives the container 90 from the catcher 511 (see FIG. 15B) of the container transfer unit 510, into the holding hole 111. In the container setting position 63, the magnet 120 magnetically collects the magnetic particles 82 in the container 90 held by the holder 110 (see FIG. 19). Thus, the magnet 120 starts the collection of the magnetic particles 82 in the container 90 from when the container 90 is set in the holder 110.

In step S3, the controller 400 causes the movement mechanism 130 to move the movement base 160 to the approach position 61. In the approach position 61, the magnet 120 magnetically collects the magnetic particles 82 in the container 90 held by the holder 110 (see FIG. 19).

In step S4, the controller 400 causes the suction tube 150 to remove the liquid component 88 in the container 90. The controller 400 causes the nozzle movement mechanism 154 to insert the suction tube 150 and the discharge tube 151 into the container 90. In a state where the magnetic particles 82 in the container 90 are magnetically collected, the controller 400 causes the suction tube 150 to suck the liquid component 88 in the container 90 (see FIG. 19).

After the suction of the liquid component 88, in step S5, the controller 400 causes the discharge tube 151 to discharge the washing liquid 55 into the container 90 in which the magnetic particles 82 are magnetically collected (see FIG. 19). After the discharge of the washing liquid 55, the controller 400 causes the nozzle movement mechanism 154 to withdraw the suction tube 150 and the discharge tube 151 from the container 90, and move them to the washer 153.

In step S6, the controller 400 causes the movement mechanism 130 to move the movement base 160 to the separate position 62. When the movement base 160 is moved to the separate position 62, the magnet 120 is separated from the holder 110 due to contact of the guide portion 170 with the rotating body 121. As a result, in the separate position 62, the magnet 120 cancels magnetic collection of the magnetic particles 82 in the container 90 held by the holder 110. In parallel with the movement of the movement base 160 to the separate position 62, the controller 400 causes the nozzle movement mechanism 154 to move the suction tube 150 into the washer 153, and causes the washer 153 to start washing of the suction tube 150.

In step S7, the controller 400 causes the agitator 140 to agitate the sample in the container 90 held by the holder 110 (see FIG. 19). The agitator 140 eccentrically rotates the holder 110. The eccentric rotation of the holder 110 vibrates the sample in the container 90, thereby agitating the magnetic particles 82 whose magnetic collection has been canceled in the separate position 62, the washing liquid 55, and unnecessary components. As a result, the magnetic particles 82 and the unnecessary components are dispersed in the washing liquid 55. During the agitation in step S7, washing of the suction tube 150 is continued in the washer 153.

In step S8, the controller 400 determines whether or not suction of the liquid component 88 and discharge of the washing liquid 55, and agitation of the sample have been repeated a predetermined number of times. For example, the predetermined number of times is 3. That is, the controller 400 determines whether or not the processes in steps S3 to S7 have been repeated 3 times (i.e., whether or not the processes in steps S3 to S7 have been executed 4 times in total). If the processes in steps S3 to S7 have not been repeated the predetermined number of times, the controller 400 proceeds the process to step S9.

In step S9, the controller 400 determines whether or not to receive another container 90 to be subjected to the BF separation process. Upon determining to receive another container 90, the controller 400 returns the process to step S1. Then, in the container setting position 63, the container 90 is transferred to a holder 110 of an empty processing port 101 in step S2. While the container 90 is being set in the empty processing port 101 in the container setting position 63, the state of magnetic collection in the container 90 being subjected to the BF separation process is maintained by the magnet 120.

Upon determining not to receive another container 90, the controller 400 returns the process to step S3, and causes suction of the liquid component 88 and discharge of the washing liquid, and agitation of the sample to be executed through steps S3 to S7. When the controller 400 has determined in step S8 that suction of the liquid component 88 and discharge of the washing liquid, and agitation of the sample have been repeated the predetermined number of times, the controller 400 proceeds the process to step S10.

In step S10, the controller 400 causes the movement mechanism 130 to move the movement base 160 to the approach position 61. In step S11, the controller 400 causes the suction tube 150 to remove the liquid component 88 in the container 90 (see FIG. 19). Thus, the magnetic particles 82 are left in the container 90, and the BF separation process on the sample in the container 90 is completed.

In step S12, the controller 400 causes the movement mechanism 130 to move the movement base 160 to the container setting position 63. In addition, the controller 400 causes the washer 153 to execute washing of the suction tube 150. In step S13, the controller 400 causes the container transfer unit 510 (see FIG. 11) to take out the container 90 for which the BF separation process has been completed, from the holder 110 of the processing port 101. The container transfer unit 510 moves the taken out container 90 to be subjected to the next process step. In the container setting position 63, while the container 90 for which the BF separation process has been completed is being taken out, the state of magnetic collection in the container 90 in the processing port 101 being subjected to the BF separation process is maintained by the magnet 120.

As described above, in the case where the BF separator 100 includes a plurality of processing ports 101, during the BF separation process performed on a certain processing port 101, a container 90 may be set in or removed from another processing port 101. In the configuration example shown in FIG. 12 to FIG. 17, since magnetic collection by the magnet 120 can be performed even in the container setting position 63, it is possible to maintain the state of magnetic collection in the container 90 in the processing port 101 being subjected to the BF separation process even during setting or removal of a container 90 in/from another processing port 101. Therefore, the BF separation process can be efficiently performed in a short time.

### (Outline of immunoassay)

In the configuration example shown in FIG. 11, as described above, immunoassay is performed by use of the R1 reagent to the R5 reagent. With reference to FIG. 20, as an example of immunoassay, a case in which the test substance 81 is hepatitis B surface antigen (HBsAg) will be described.

First, a specimen containing the test substance 81, and the R1 reagent are dispensed into a container 90. The first reagent dispenser 551 dispenses the R1 reagent into the container 90, and the specimen dispensing unit 530 dispenses the specimen into the container 90. The R1 reagent contains a capture substance 84, and reacts with the test substance 81 to be bound thereto. The capture substance 84 contains a binding substance for allowing the capture substance 84 to bind to the magnetic particles 82 contained in the R2 reagent.

For example, the capture substance 84 is an antibody modified with biotin (biotin antibody). That is, the capture substance 84 is modified with biotin as the binding substance. After dispensing of the specimen and the R1 reagent, the sample in the container 90 is heated at a predetermined temperature in the reaction unit 580, whereby the capture substance 84 binds to the test substance 81.

Next, the second reagent dispenser 552 dispenses the R2 reagent into the container 90. The R2 reagent contains the magnetic particles 82. The magnetic particle 82 binds to the binding substance of the capture substance 84. The magnetic particle 82 is a magnetic particle having immobilized thereon streptavidin which binds to biotin (StAvi-bound magnetic particle). Streptavidin of the StAvi-bound magnetic particle reacts with biotin as the binding substance, and binds to biotin. After dispensing of the R2 reagent, the sample in the container 90 is heated at a predetermined temperature in the reaction unit 580. As a result, the test substance 81 and the capture substance 84 bind to the magnetic particle 82.

The test substance 81 and the capture substance 84 formed on the magnetic particle 82 are separated from unreacted capture substance 84, through a primary BF separation process (see FIG. 18, FIG. 19) by the BF separator 100. Through the primary BF separation process, unnecessary components such as the unreacted capture substance 84 are removed from the container 90.

Next, the third reagent dispenser 553 dispenses the R3 reagent into the container 90. The R3 reagent contains the labeled substance 83, and reacts with the test substance 81 to be bound thereto. After dispensing of the R3 reagent, the sample in the container 90 is heated at a predetermined temperature in the reaction unit 580. As a result, an immune complex 85 containing the test substance 81, the labeled substance 83, and the capture substance 84 is formed on the magnetic particle 82. In the example shown in FIG. 20, the labeled substance 83 is an ALP (alkaline phosphatase) labeled antibody.

The immune complex 85 formed on the magnetic particle 82 is separated from unreacted labeled substance 83, through a secondary BF separation process (see FIG. 18, FIG. 19) by the BF separator 100. Through the secondary BF separation process, unnecessary components such as the unreacted labeled substance 83 are removed from the container 90.

Thereafter, the fourth reagent dispenser 554 and the fifth reagent dispenser 555 dispense the R4 reagent and the R5 reagent into the container 90, respectively. The R4 reagent contains a buffer. The immune complex 85 bound to the magnetic particle 82 is dispersed in the buffer. The R5 reagent contains a chemiluminescent substrate. The buffer contained in the R4 reagent has a composition that facilitates reaction between the substrate and the label (enzyme) of the labeled substance 83 contained in the immune complex 85. After dispensing of the R4 and R5 reagents, the sample in the container 90 is heated at a predetermined temperature in the reaction unit 580. Light is generated as a result of reaction between the substrate and the label, and the intensity of the generated light is measured by the photodetector 521 in the detection unit 520. Based on a detection signal from the detection unit 520, the controller 400 analyzes, for example, the content of the test substance 81 in the specimen.

### (Description of analysis operation)

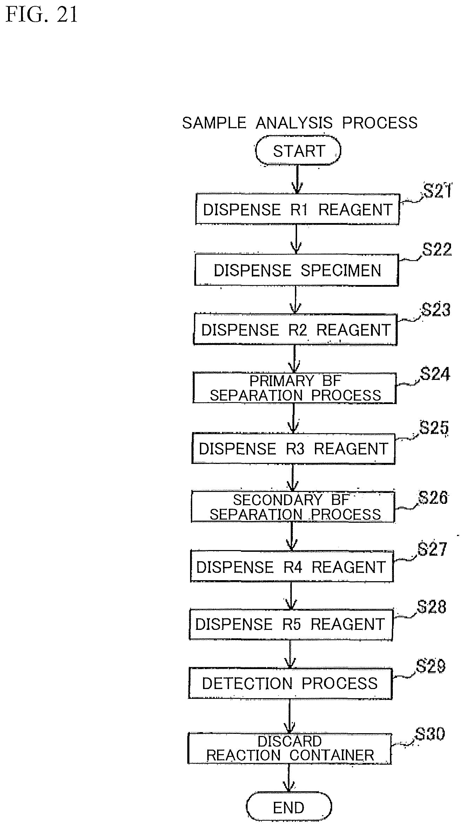

Next, an analysis operation of the sample analyzer 500 shown in FIG. 20 will be described with reference to FIG. 21. The process in each step shown in FIG. 21 is controlled by the controller 400.

In step S21, the controller 400 causes the container transfer unit 510 to transfer the container 90 to the R1 reagent dispensing position. The controller 400 causes the first reagent dispenser 551 to dispense the R1 reagent into the container 90.

In step S22, the specimen is dispensed into the container 90. The controller 400 causes the specimen dispensing unit 530 to suck the specimen from the test tube on the specimen transport unit 560. The controller 400 causes the specimen dispensing unit 530 to dispense the sucked specimen into the container 90. Each time the specimen dispensing unit 530 has performed a dispensing operation via a dispensing tip, the specimen dispensing unit 530 replaces the dispensing tip with an unused dispensing tip.

In step S23, the controller 400 causes the container transfer unit 510 to transfer the container 90 to the R2 reagent dispensing position, and causes the second reagent dispenser 552 to dispense the R2 reagent into the container 90. After dispensing of the R2 reagent, the controller 400 causes the container transfer unit 510 to transfer the container 90 to the reaction unit 580. The container 90 is heated for a predetermined time in the reaction unit 580.

In step S24, the controller 400 causes the BF separator 100 to execute the primary BF separation process. First, the controller 400 causes the container transfer unit 510 to transfer the container 90 to the BF separator 100. The BF separator 100 is controlled to perform the primary BF separation process on the sample in the container 90.

In step S25, the controller 400 causes the container transfer unit 510 to transfer the container 90 to the R3 reagent dispensing position, and causes the third reagent dispenser 553 to dispense the R3 reagent into the container 90. After dispensing of the R3 reagent, the controller 400 causes the container transfer unit 510 to transfer the container 90 to the reaction unit 580. The container 90 is heated for a predetermined time in the reaction unit 580.

In step S26, the controller 400 causes the BF separator 100 to execute the secondary BF separation process. First, the controller 400 causes the container transfer unit 510 to transfer the container 90 to the BF separator 100. The BF separator 100 is controlled to perform the secondary BF separation process on the sample in the container 90.

In step S27, the R4 reagent is dispensed into the container 90. The controller 400 causes the container transfer unit 510 to transfer the container 90 to the R4 reagent dispensing position, and causes the fourth reagent dispenser 554 to dispense the R4 reagent into the container 90.

In step S28, the R5 reagent is dispensed into the container 90. The controller 400 causes the container transfer unit 510 to transfer the container 90 to the R5 reagent dispensing position, and causes the fifth reagent dispenser 555 to dispense the R5 reagent into the container 90. After dispensing of the R5 reagent, the controller 400 causes the container transfer unit 510 to transfer the container 90 to the reaction unit 580. The container 90 is heated for a predetermined time in the reaction unit 580.

In step S29, a detection process for the immune complex 85 is performed. The controller 400 causes the container transfer unit 510 to transfer the container 90 to the detection unit 520. The detection unit 520 measures the intensity of light generated as a result of reaction between the substrate and the label. A detection result from the detection unit 520 is outputted to the controller 400.

After the detection has ended, in step S30, the container transfer unit 510 is controlled to take out the container 90 having been subjected to the analysis process from the detection unit 520, and discard the container 90 into a discard hole (not shown).

In this manner, the analysis operation of the sample analyzer 500 is performed.

It should be noted that the embodiments disclosed herein are merely illustrative in all aspects and should not be considered as restrictive. The scope of the present invention is defined not by the description of the above-described embodiments but by the scope of the claims, and includes meaning equivalent to the scope of the claims and all changes within the scope.

### INDUSTRIAL APPLICABILITY

The BF separator, the sample analyzer, and the BF separation method according to the present invention can be suitably used in the field of immunoassays.

### DESCRIPTION OF THE REFERENCE CHARACTERS

- 10, 110: holder
- 11, 111: holding hole
- 20, 120: magnet
- 30, 130: movement mechanism
- 31, 170: guide portion
- 40, 140: agitator
- 50, 150: suction tube
- 51, 151: discharge tube
- 55: washing liquid
- 61: approach position
- 62: separate position
- 63: container setting position
- 81: test substance
- 82: magnetic particle
- 83: labeled substance
- 88: liquid component
- 90: container
- 100: BF separator
- 101: processing port
- 113: open side surface
- 114: opening
- 121: rotating body
- 125: urging member
- 131: drive unit
- 153: washer
- 160: movement base
- 170, 170a, 170b: inclined surface
- 500: movement base
- 510: container transfer unit
- 520: detection unit
- D1: distance that allows magnetic collection
- D2: distance that allows cancellation of magnetic collection

## Claims

1. ABF separator comprising:
a holder configured to hold a container that stores therein a sample containing magnetic particles and a liquid component;
a magnet provided to be movable relative to the holder in a direction approaching or separating from the holder, and configured to magnetically collect the magnetic particles;
a movement mechanism configured to move the holder, and move the magnet in conjunction with movement of the holder, in a direction in which the holder and the magnet approach each other or separate from each other;
an agitator configured to agitate the sample in the container held by the holder located in a separate position where the holder and the magnet are away from each other; and
a suction tube configured to suck the liquid component in the container held by the holder located in an approach position where the holder and the magnet are close to each other.

2. The BF separator according to claim 1, wherein the approach position and the separate position are different positions in a height direction.

3. The BF separator according to claim 1, further comprising a discharge tube configured to discharge a washing liquid into the container held by the holder located in the approach position, wherein
the agitator agitates the sample, in the container, containing the magnetic particle and the washing liquid.

4. The BF separator according to claim 3, wherein the movement mechanism moves the holder and the magnet to the approach position and to the separate position, a predetermined number of times, so as to repeat: suction of the liquid component by the suction tube and discharge of the washing liquid by the discharge tube in the approach position; and agitation of the sample by the agitator in the separate position.

5. The BF separator according to claim 1, wherein
in the approach position, the holder and the magnet are close to each other over a distance that allows the magnetic particles in the container to be magnetically collected, and
in the separate position, the holder and the magnet are away from each other over a distance that allows magnetic collection of the magnetic particles in the container to be canceled.

6. The BF separator according to claim 1, wherein the agitator is connected to the holder so as to move together with the holder, and is configured to agitate the sample in the container by driving the holder.

7. The BF separator according to claim 1, wherein
the movement mechanism is configured to move the holder and the magnet in an up-down direction, and
the holder and the magnet relatively move so as to approach each other or separate from each other in a direction intersecting the up-down direction, in conjunction with the movement in the up-down direction.

8. The BF separator according to claim 1, further comprising a guide portion configured to come into contact with at least one of the holder and the magnet being moved by the movement mechanism, and cause the holder and the magnet to approach each other or separate from each other.

9. The BF separator according to claim 8, further comprising a biasing member configured to bias the holder and the magnet in a direction in which the holder and the magnet approach each other, wherein
the guide portion is configured to cause the holder and the magnet to separate from each other against a biasing force of the biasing member, and cause the holder and the magnet to approach each other by the biasing force of the biasing member.

10. The BF separator according to claim 8, wherein
the guide portion has a surface that is inclined with respect to a movement direction from the approach position to the separate position such that the holder and the magnet are separated from each other as the guide portion approaches from the approach position to the separate position, and
at least one of the holder and the magnet includes a rotating body that rolls on the inclined surface.

11. The BF separator according to claim 1, wherein the magnet is disposed laterally to the holder so as to approach or separate from a side surface of the container held by the holder.

12. The BF separator according to claim 11, wherein the holder is configured to expose the side surface, on the magnet side, of the held container, such that the magnet and the container held by the holder are close to each other in the approach positon.

13. The BF separator according to claim 12, wherein
the holder has a holding hole for holding the container, and an open side surface having, formed therein, an opening that exposes the side surface of the container disposed in the holding hole, and
in the approach position, the magnet is disposed so as to be in contact with the open side surface.

14. The BF separator according to claim 1, wherein the approach position and the separate position are positions that allow at least one of the holder and the magnet to be disposed on a straight line in the up-down direction.

15. The BF separator according to claim 1, wherein the approach position is located below the separate position.

16. The BF separator according to claim 15, further comprising a washer which is disposed at substantially the same height position as the holder in the approach position, and is configured to wash the suction tube, wherein
the suction tube is configured to move between the container held by the holder in the approach position, and the washer.

17. The BF separator according to claim 1, wherein the movement mechanism is configured to move the holder to a container setting position where setting and removal of the container are performed.

18. The BF separator according to claim 17, wherein, in the container setting position, the holder and the magnet are configured to approach each other over a distance that allows the magnetic particles in the container to be magnetically collected.

19. The BF separator according to claim 17, wherein the container setting position is located above the approach position and the separate position.

20. The BF separator according to claim 1, wherein the movement mechanism is configured to move the holder, the magnet, and the agitator in conjunction with one another.

21. The BF separator according to claim 1, wherein the movement mechanism includes a common drive unit configured to move both the holder and the magnet.

22. The BF separator according to claim 21, further comprising a movement base on which the holder and the magnet are provided, wherein
the movement mechanism causes the drive unit to move the movement base.

23. The BF separator according to claim 22, wherein the agitator is provided on the movement base, and is configured to agitate the sample in the container when the holder is driven.

24. The BF separator according to claim 22, wherein a plurality of processing ports each including the holder and the magnet are provided on the movement base.

25. A sample analyzer comprising:
a container transfer unit configured to transfer a container that stores therein a sample containing a test substance, magnetic particles that bind to the test substance, and a labeled substance that binds to the test substance;
a BF separator according to claim 1, configured to separate the magnetic particles from a liquid component in the sample in the container transferred by the container transfer unit; and
a detection unit configured to detect the test substance bound to the magnetic particles in the container from which the liquid component is separated by the BF separator.

26. ABF separation method comprising:
moving a holder holding a container that stores therein a sample containing magnetic particles and a liquid component, and causing a magnet to approach the holder in conjunction with the movement of the holder;
sucking the liquid component in the container held by the holder located in an approach position where the holder and the magnet are close to each other;
discharging a washing liquid into the container from which the liquid component has been sucked;
moving the holder from the approach position, and causing the magnet to separate from the holder in conjunction with the movement of the holder; and
agitating the sample in the container held by the holder located in a separate position where the holder and the magnet are away from each other.
